(19) Europäisches Patentamt

European Patent Office

Office européen des brevets

(11) **EP 1 298 205 A1**

(12) **EUROPEAN PATENT APPLICATION**
published in accordance with Art. 158(3) EPC

(43) Date of publication:
02.04.2003 Bulletin 2003/14

(51) Int Cl.7: **C12N 9/16**, C12N 15/55, C07K 16/40, A61K 38/43

(21) Application number: 00942470.6

(22) Date of filing: 03.07.2000

(86) International application number:
**PCT/JP00/04441**

(87) International publication number:
**WO 02/002762 (10.01.2002 Gazette 2002/02)**

(84) Designated Contracting States:
**AT BE CH CY DE DK ES FI FR GB GR IE IT LI LU MC NL PT SE**

(71) Applicant: **MOCHIDA PHARMACEUTICAL CO., LTD.**
**Shinjuku-ku Tokyo 160-8515 (JP)**

(72) Inventors:
• **INOUE, Keizo**
**Hannou-shi, Saitama 357-0041 (JP)**

• **ARAI, Hiroyuki, 604 Kurea Homes**
**Tokyo 112-0002 (JP)**
• **AOKI, Junken,**
**306 2goto Nakagin Setagaya Mansion**
**Tokyo 154-0002 (JP)**

(74) Representative: **Krauss, Jan**
**Forrester & Boehmert,**
**Pettenkoferstrasse 20-22**
**80336 München (DE)**

(54) **NOVEL LIPASE**

(57) **(57) Abstract:** A novel phospholipase $A_1$ ($PLA_1$) having a substrate specificity to phosphatidic acid (PA); a peptide or a polypeptide originating in the above novel $PLA_1$; a polynucleotide encoding the peptide or polypeptide originating in the novel $PLA_1$; a process for producing the peptide or polypeptide originating in the novel $PLA_1$; an antibody against the peptide or polypeptide originating in the novel $PLA_1$; a method of identifying an inhibitor, an antagonist or an activator of the novel $PLA_1$ by using the same; compounds identified by the above method; and medicinal compositions and diagnostic methods by using the same.

EP 1 298 205 A1

SEQUENCE LISTING

<110> Mochida Pharmaceutical Co. Ltd.

<120> A novel lipase

<130> GP00-1011

<150> JP P1999-187089
<151> 1999-06-30

<160> 8

<170> PatentIn version 3.1

<210> 1
<211> 2445
<212> DNA
<213> Homo sapiens

<220>
<221> CDS
<222> (89)..(1441)
<223> Sequence number 2 described in the specification

<220>
<221> misc_feature
<223> "<210> 1" is Sequence number 2 described in the specification.
      "<210> 2" is Sequence number 1 described in the specification.

<400> 1
cacgagaaaa tcccacagtg gaaactctta agcctctgcg aagtaaatca ttcttgtgaa      60

tgtgacacac gatctctcca gtttccat atg ttg aga ttc tac tta ttc atc      112
                            Met Leu Arg Phe Tyr Leu Phe Ile
                              1               5

agt ttg ttg tgc ttg tca aga tca gac gca gaa gaa aca tgt cct tca      160
Ser Leu Leu Cys Leu Ser Arg Ser Asp Ala Glu Glu Thr Cys Pro Ser
       10              15              20

ttc acc agg ctg agc ttt cac agt gca gtg gtt ggt acg gga cta aat      208
Phe Thr Arg Leu Ser Phe His Ser Ala Val Val Gly Thr Gly Leu Asn
25              30              35              40

gtg agg ctg atg ctc tac aca agg aaa aac ctg acc tgc gca caa acc      256
Val Arg Leu Met Leu Tyr Thr Arg Lys Asn Leu Thr Cys Ala Gln Thr
               45              50              55

atc aac tcc tca gct ttt ggg aac ttg aat gtg acc aag aaa acc acc      304
Ile Asn Ser Ser Ala Phe Gly Asn Leu Asn Val Thr Lys Lys Thr Thr

15

```
                    60                      65                      70

ttc att gtc cat gga ttc agg cca aca ggc tcc cct cct gtt tgg atg        352
Phe Ile Val His Gly Phe Arg Pro Thr Gly Ser Pro Pro Val Trp Met
        75                      80                      85

gat gac tta gta aag ggt ttg ctc tct gtt gaa gac atg aac gta gtt        400
Asp Asp Leu Val Lys Gly Leu Leu Ser Val Glu Asp Met Asn Val Val
        90                      95                      100

gtt gtt gat tgg aat cga gga gct aca act tta ata tat acc cat gcc        448
Val Val Asp Trp Asn Arg Gly Ala Thr Thr Leu Ile Tyr Thr His Ala
105                     110                     115                 120

tct agt aag acc aga aaa gta gcc atg gtc ttg aag gaa ttt att gac        496
Ser Ser Lys Thr Arg Lys Val Ala Met Val Leu Lys Glu Phe Ile Asp
                    125                     130                     135

cag atg ttg gca gaa gga gct tct ctt gat gac att tac atg atc gga        544
Gln Met Leu Ala Glu Gly Ala Ser Leu Asp Asp Ile Tyr Met Ile Gly
                140                     145                     150

gta agt cta gga gcc cac ata tct ggg ttt gtt gga gag atg tac gat        592
Val Ser Leu Gly Ala His Ile Ser Gly Phe Val Gly Glu Met Tyr Asp
                155                     160                     165

gga tgg ctg ggg aga att aca ggc ctc gac cct gca ggc cct tta ttc        640
Gly Trp Leu Gly Arg Ile Thr Gly Leu Asp Pro Ala Gly Pro Leu Phe
                170                     175                     180

aac ggg aaa cct cac caa gac aga tta gat ccc agt gat gcg cag ttt        688
Asn Gly Lys Pro His Gln Asp Arg Leu Asp Pro Ser Asp Ala Gln Phe
185                     190                     195                 200

gtt gat gtc atc cat tcc gac act gat gca ctg ggc tac aag gag cca        736
Val Asp Val Ile His Ser Asp Thr Asp Ala Leu Gly Tyr Lys Glu Pro
                    205                     210                     215

tta gga aac ata gac ttc tac cca aat gga gga ttg gat caa cct ggc        784
Leu Gly Asn Ile Asp Phe Tyr Pro Asn Gly Gly Leu Asp Gln Pro Gly
                220                     225                     230

tgc ccc aaa aca ata ttg gga gga ttt cag tat ttt aaa tgt gac cac        832
Cys Pro Lys Thr Ile Leu Gly Gly Phe Gln Tyr Phe Lys Cys Asp His
                235                     240                     245

cag agg tct gta tac ctg tac ctg tct tcc ctg aga gag agc tgc acc        880
Gln Arg Ser Val Tyr Leu Tyr Leu Ser Ser Leu Arg Glu Ser Cys Thr
                250                     255                     260

atc act gcg tat ccc tgt gac tcc tac cag gat tat agg aat ggc aag        928
Ile Thr Ala Tyr Pro Cys Asp Ser Tyr Gln Asp Tyr Arg Asn Gly Lys
265                     270                     275                 280
```

```
tgt gtc agc tgc ggc acg tca caa aaa gag tcc tgt ccc ctt ctg ggc    976
Cys Val Ser Cys Gly Thr Ser Gln Lys Glu Ser Cys Pro Leu Leu Gly
              285             290             295

tat tat gct gat aat tgg aaa gac cat cta agg ggg aaa gat cct cca    1024
Tyr Tyr Ala Asp Asn Trp Lys Asp His Leu Arg Gly Lys Asp Pro Pro
              300             305             310

atg acg aag gca ttc ttt gac aca gct gag gag agc cca ttc tgc atg    1072
Met Thr Lys Ala Phe Phe Asp Thr Ala Glu Glu Ser Pro Phe Cys Met
              315             320             325

tat cat tac ttt gtg gat att ata aca tgg aac aag aat gta aga aga    1120
Tyr His Tyr Phe Val Asp Ile Ile Thr Trp Asn Lys Asn Val Arg Arg
              330             335             340

ggg gac att acc atc aaa ttg aga gac aaa gct gga aac acc aca gaa    1168
Gly Asp Ile Thr Ile Lys Leu Arg Asp Lys Ala Gly Asn Thr Thr Glu
345             350             355             360

tcc aaa atc aat cat gaa ccc acc aca ttt cag aaa tat cac caa gtg    1216
Ser Lys Ile Asn His Glu Pro Thr Thr Phe Gln Lys Tyr His Gln Val
              365             370             375

agt cta ctt gca aga ttt aat caa gat ctg gat aaa gtg gct gca att    1264
Ser Leu Leu Ala Arg Phe Asn Gln Asp Leu Asp Lys Val Ala Ala Ile
              380             385             390

tcc ttg atg ttc tct aca gga tct cta ata ggc cca agg tac aag ctc    1312
Ser Leu Met Phe Ser Thr Gly Ser Leu Ile Gly Pro Arg Tyr Lys Leu
              395             400             405

agg att ctc cga atg aag tta agg tcc ctt gcc cat ccg gag agg cct    1360
Arg Ile Leu Arg Met Lys Leu Arg Ser Leu Ala His Pro Glu Arg Pro
              410             415             420

cag ctg tgt cgg tat gat ctt gtc ctg atg gaa aac gtt gaa aca gtc    1408
Gln Leu Cys Arg Tyr Asp Leu Val Leu Met Glu Asn Val Glu Thr Val
425             430             435             440

ttc caa cct att ctt tgc cca gag ttg cag ttg taactgttgc caggacacat  1461
Phe Gln Pro Ile Leu Cys Pro Glu Leu Gln Leu
              445             450

ggccataaat aatagaaaga aagctacaac cacaggctgt ttgaaagctt cacctcacct  1521

ttctgcaaag cagaaaaagt atgaaaaaac caaggctttt ttcagtagcg tcctatggat  1581

gtcacattgt acatcaaaca accttgtgat tataaaacga tcctgggaag gagcccctaa  1641

ctagggcaag tcagaaatag ccaggctcgc agcagcgcag cgctgtgtct gctgtgtcct  1701
```

```
ggggcctccc ttgttccgac ctgtcaattc tgctgcctgt cacgcgggtg gttctgccca    1761

tcgcggctgc gggtcaagca tcttcaaggg aaggacggac tggaggcctc accgtggact    1821

caactctgca ttctccgtgc cacattcctc cagttcccac acgtagaagg gaacgaaact    1881

gacgtctacc tcatggggct gctgtgtggg tttgggaggc aaaaatctat gaagggtttt    1941

ttgaaatccc ataggtgcca catctatgag atgtttgata aatgtgaata tgcttttaca    2001

tttgggctta tctaatttgc aataagagag cctctctcta tcaacaccag cttctctctc    2061

gggctgtttg ctcagggaag gcaagaaagc cacgtgctgg ccctctgcct tctctaaagt    2121

gctgttggag catggaggag ctggaggaga tggggatgga ctgacagcta agagggcggc    2181

tgctgggact agatagtgga tgaagaaaga aggacgagga agccgtgggg cagcctcttc    2241

acatggggac aggggatgga gcatgaggca ggggaaggaa aagcagagct tatttttcac    2301

ctaaggtgga gaaggatcac tttacaggca acgctcattt taagcaaccc ttaagaaatg    2361

tttatgtttc tttattacca atgtaatcta tgattattga aggaaattta gaaaatgcgt    2421

agatacaaaa aaaaaaaaaa aaaa    2445
```

<210> 2
<211> 451
<212> PRT
<213> Homo sapiens

<400> 2

```
Met Leu Arg Phe Tyr Leu Phe Ile Ser Leu Leu Cys Leu Ser Arg Ser
1               5                   10                  15


Asp Ala Glu Glu Thr Cys Pro Ser Phe Thr Arg Leu Ser Phe His Ser
            20                  25                  30


Ala Val Val Gly Thr Gly Leu Asn Val Arg Leu Met Leu Tyr Thr Arg
            35                  40                  45


Lys Asn Leu Thr Cys Ala Gln Thr Ile Asn Ser Ser Ala Phe Gly Asn
        50                  55                  60


Leu Asn Val Thr Lys Lys Thr Thr Phe Ile Val His Gly Phe Arg Pro
65                  70                  75                  80
```

```
Thr Gly Ser Pro Pro Val Trp Met Asp Asp Leu Val Lys Gly Leu Leu
                85              90              95

Ser Val Glu Asp Met Asn Val Val Val Asp Trp Asn Arg Gly Ala
            100             105             110

Thr Thr Leu Ile Tyr Thr His Ala Ser Ser Lys Thr Arg Lys Val Ala
        115             120             125

Met Val Leu Lys Glu Phe Ile Asp Gln Met Leu Ala Glu Gly Ala Ser
    130             135             140

Leu Asp Asp Ile Tyr Met Ile Gly Val Ser Leu Gly Ala His Ile Ser
145             150             155             160

Gly Phe Val Gly Glu Met Tyr Asp Gly Trp Leu Gly Arg Ile Thr Gly
            165             170             175

Leu Asp Pro Ala Gly Pro Leu Phe Asn Gly Lys Pro His Gln Asp Arg
        180             185             190

Leu Asp Pro Ser Asp Ala Gln Phe Val Asp Val Ile His Ser Asp Thr
        195             200             205

Asp Ala Leu Gly Tyr Lys Glu Pro Leu Gly Asn Ile Asp Phe Tyr Pro
    210             215             220

Asn Gly Gly Leu Asp Gln Pro Gly Cys Pro Lys Thr Ile Leu Gly Gly
225             230             235             240

Phe Gln Tyr Phe Lys Cys Asp His Gln Arg Ser Val Tyr Leu Tyr Leu
            245             250             255

Ser Ser Leu Arg Glu Ser Cys Thr Ile Thr Ala Tyr Pro Cys Asp Ser
        260             265             270

Tyr Gln Asp Tyr Arg Asn Gly Lys Cys Val Ser Cys Gly Thr Ser Gln
        275             280             285
```

```
Lys Glu Ser Cys Pro Leu Leu Gly Tyr Tyr Ala Asp Asn Trp Lys Asp
    290                 295                 300

His Leu Arg Gly Lys Asp Pro Pro Met Thr Lys Ala Phe Phe Asp Thr
305                 310                 315                 320

Ala Glu Glu Ser Pro Phe Cys Met Tyr His Tyr Phe Val Asp Ile Ile
                325                 330                 335

Thr Trp Asn Lys Asn Val Arg Arg Gly Asp Ile Thr Ile Lys Leu Arg
                340                 345                 350

Asp Lys Ala Gly Asn Thr Thr Glu Ser Lys Ile Asn His Glu Pro Thr
        355                 360                 365

Thr Phe Gln Lys Tyr His Gln Val Ser Leu Leu Ala Arg Phe Asn Gln
    370                 375                 380

Asp Leu Asp Lys Val Ala Ala Ile Ser Leu Met Phe Ser Thr Gly Ser
385                 390                 395                 400

Leu Ile Gly Pro Arg Tyr Lys Leu Arg Ile Leu Arg Met Lys Leu Arg
                405                 410                 415

Ser Leu Ala His Pro Glu Arg Pro Gln Leu Cys Arg Tyr Asp Leu Val
                420                 425                 430

Leu Met Glu Asn Val Glu Thr Val Phe Gln Pro Ile Leu Cys Pro Glu
                435                 440                 445

Leu Gln Leu
    450


<210>  3
<211>  24
<212>  DNA
<213>  Homo sapiens

<400>  3
tgcgaagtaa atcattcttg tgaa                                          24
```

<210> 4
<211> 24
<212> DNA
<213> Homo sapiens

<400> 4
tgtgacatcc ataggacgct actg                                                      24


<210> 5
<211> 24
<212> DNA
<213> Homo sapiens

<400> 5
ctgcgcacaa accatcaact cctc                                                      24


<210> 6
<211> 24
<212> DNA
<213> Homo sapiens

<400> 6
aggggacagg actctttttg tgac                                                      24


<210> 7
<211> 33
<212> DNA
<213> Homo sapiens

<400> 7
cgcggatcca tgttgagatt ctacttattc atc                                            33


<210> 8
<211> 60
<212> DNA
<213> Homo sapiens

<400> 8
ccggaattct tacttgtcat cgtcgtcctt gtagtccaac tgcaactctg ggcaaagaat              60


.

## Description

### Technical Field

The present invention relates to a novel lipase, particularly phospholipase $A_1$ (hereafter sometimes $PLA_1$). More specifically, a peptide or polypeptide comprising all or a portion of an amino acid sequence of the novel $PLA_1$; a polynucleotide encoding the peptide or the polypeptide; a recombinant vector comprising the polynucleotide; a transformant transformed by the recombinant vector; a method for producing the peptide or the polypeptide by using the transformant; an antibody against the peptide or the polypeptide; a method for identifying a compound by using the above materials; the compound identified; an inhibitor or an activator, which acts on the polypeptide or the polynucleotide; a medicinal composition related to the same and a method for producing thereof; a method of treatment by using the medicinal composition; and the method for diagnosing a disease, which is related to $PLA_1$.

### Background Art

$PLA_1$ is an enzyme which hydrolyzes an ester linkage in a first position of glycerol of a glycerophospholipid. To date, the presence of such an enzyme activity has been detected in various organs and some types of $PLA_1$ which are distinguishable in accordance with their substrate specificity have been reported. Examples of such enzymes whose cDNA has been cloned include toxin $PLA_1$ (Dolm1), $PS-PLA_1$ [which specifically hydrolyzes the ester linkage in the first position of glycerol of phosphatidyl serine (PS) and lysophosphatidyl serine (lysoPS) (JP H10-201479 A) (Protein, Nucleic Acid, and Enzyme 44: 1038 - 1042, 1999)] and $PA-PLA_1$ from human testes [which specifically hydrolyzes the ester linkage in the first position of glycerol of phosphatidic acid (PA) (J. Biol. Chem. 273: 5468 - 5477, 1998)]. Moreover, it has been known that many molecules of the lipase family have triacylglycerol decomposing activity and also $PLA_1$ activity (FEBS Letters 320: 145 - 149, 1993), (Biochemistry 32: 4702 - 4707, 1993) and (J. Biol. Chem. 272: 2192 - 2198, 1997). In addition, $PLA_1$'s belonging to the lipase family found to date all have a short lid (B.B.A. 1376:417-432, 1998) and (Biochemistry 32: 4702 - 4707, 1993). The physiological role of the lid has not been clearly identified . On the other hand, it was suggested that there is a possibility of involvement in the lipase activity of "a sugar chain of a lipase molecule" (J. Lipid Res. 35: 1511 - 1523, 1994) and (J. Lipid Res. 36: 939 - 951, 1995).

One of functions of $PLA_1$ is the action to decompose a phospholipid. It has been known that lysophosphatidic acid (hereafter sometimes LPA) (B. B. A. 1198: 185 - 196, 1994), one product of the decomposition, has many physiological activities and this acid has attracted attention in terms of its biological usefulness (Cell Technology 17 (5): 739 - 745, 1998). Major actions reported for LPA include increase in blood pressure (Lipids 13: 572 - 574, 1978), platelet aggregation action (Am. J. Pathol. 96: 423 - 438, 1979), cell growth promoting action (Cell 59: 45 - 54, 1989), and also cancer cell infiltration action, cell adhesion, stress fiber formation, chemotaxis induction, neural spine retraction, apoptosis suppression, and involvement in wound healing (B. B. A. 1198: 185 - 196, 1994).

As $PLA_1$ having phosphatidic acid (hereafter, sometimes PA) specificity, the human testes $PA-PLA_1$ has been known and a cDNA thereof has been cloned. The novel $PLA_1$ is an enzyme inside a cell and is considered to be a factor which determines metabolic turnover of a fatty acid in an sn-1 position of phosphatidic acid, which is the center of phospholipid metabolism (J. Biol. Chem. 273: 5468 - 5477, 1998). On the other hand, it has been reported that the human testes $PA-PLA_1$ hydrolyses phosphatidyl ethanolamine (PE) and phosphatidyl inositol (PI) in accordance with the reaction condition.

It is an object of the present invention to find a novel substance related to $PLA_1$ which is a catalyst for producing LPA, and which is a causal substance of the above-described various malignant symptoms, and also to control LPA in a living body.

### Disclosure of the Invention

The present invention includes:

(1) A polypeptide selected from the following groups;

(a) a polypeptide consisting of the amino acid sequence of SEQ ID NO:1,
(b) a polypeptide comprising the polypeptide of (a),
(c) a polypeptide having at least 70% amino acid sequence homology with the polypeptide of (a) and having phosphatidic acid decomposing activity, and
(4d a polypeptide having variation of one or several amino acids in the amino acid sequence of SEQ ID NO: 1, such as deletion, substitution, addition, and insertion in the amino acid sequence, and having phosphatidic acid decomposing activity,

(2) A peptide comprising at least about 8 consecutive amino acids of the amino acid sequence of SEQ ID NO:1,

(3) A polynucleotide encoding the polypeptide of (1) or the peptide of (2) or a complementary chain thereof,

(4) A polynucleotide which hybridizes to the polynucleotide of (3) under stringent conditions, and a complementary chain thereof,

(5) A polynucleotide comprising at least about 15 consecutive nucleotide bases of the polynucleotide of SEQ ID NO:2, and a complementary chain thereof,

(6) A recombinant vector comprising any one of the polynucleotides of (3) to (5),

(7) A transformant transformed by the recombinant vector of (6),

(8) A method for producing the polypeptide or a peptide of (1) or (2), comprising culturing the transformant of (7),

(9) An antibody which specifically binds the polypeptide of (1) or the peptide of (2),

(10) The antibody of (9), wherein said antibody is able to suppress phosphatidic acid decomposing activity,

(11) A method for identifying a compound which interacts with the polypeptide of (1) so as to inhibit or activate the activity of said polypeptide and/ or a compound which interacts with the polynucleotide of (3) or (4) so as to inhibit or accelerate expression thereof, wherein the method comprises a step using at least any one of the polypeptide of (1), the peptide of (2), polynucleotides of (3) to (5), the vector of (6), the transformant of (7), and the antibody of (9) or (10),

(12) A method for identifying a compound which interacts with the polypeptide of (1) so as to inhibit or activate the activity of said polypeptide and/or a compound which interacts with the polynucleotide of (3) or (4) so as to inhibit or accelerate expression thereof, wherein the method comprises the steps of evaluating the interaction with the compound by contacting the polypeptide or the polynucleotide with the compound to be screened under conditions allowing an interaction of the compound with the polypeptide or the polynucleotide (such interaction is related to a second component capable of providing a detectable signal responding to the interaction of the compound with the polypeptide or the polynucleotide) and then, determining whether the compound interacts with the polypeptide or the polynucleotide to activate or inhibit the activities thereof by detecting the presence or absence or a change thereof of the signal generated by the interaction of the compound with the polypeptide or the polynucleotide,

(13) A method for identifying a compound which inhibits or activates the activity or a physiological action of the polypeptide of (1) or the polynucleotide of (3) or (4), wherein the method comprises the steps of evaluating the interaction with the compound, by using the transformant of (7) and another transformant, which is produced by expressing a receptor for lysophosphatidic acid produced using the polypeptide of (1) and expressed in the transformant, to phosphatidic acid, by contacting these transformants with the compound to be screened under conditions allowing for interaction of the compound with the transformants (such interaction is related to the second component capable of providing a detectable signal responding to the interaction of the compound with the transformant) and then, detecting the presence or absence or a change thereof of the signal generated by the interaction of the compound with the transformant to determine whether the compound activates or inhibits the activity or the physiological action of the polypeptide or the polynucleotide,

(14) A compound identified by the methods of (11) to (13),

(15) A compound which interacts with the polypeptide of (1) so as to inhibit or activate the activity thereof, or a compound which interacts with the polynucleotide of (3) or (4) so as to inhibit or accelerate the expression thereof,

(16) A medicinal composition comprising at least any one of the polypeptide of (1) or the peptide of (2), any one of the polynucleotides of (3) to (5), the vector of (6), the transformant of (7), the antibody of (9) or (10), and the compound of (14) or (15),

(17) A method for diagnosing a disease related to expression or the activity of the polypeptide of (1) in a subject , wherein the method comprises the step of performing an analysis by using (a) a nucleic acid sequence encoding the polypeptide and/or (b) the polypeptide, contained in a sample obtained from the subject as a marker,

(18) A method for therapeutic treatment comprising administering the medicinal composition of (16) to a subject afflicted with a disease related to phospholipase $A_1$, and

(19) A method for producing the medicinal composition of (16).

**Brief Description of the Drawings**

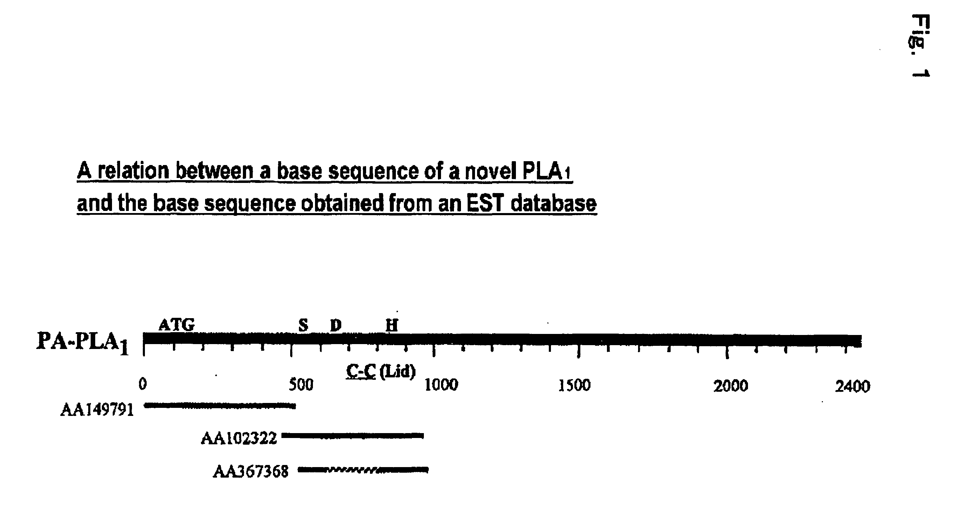

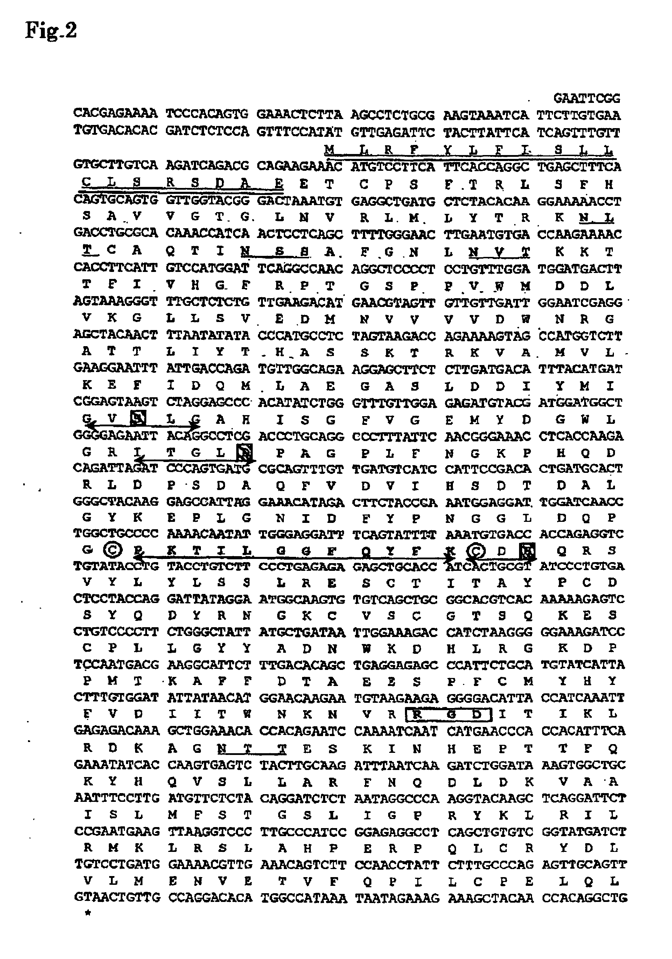

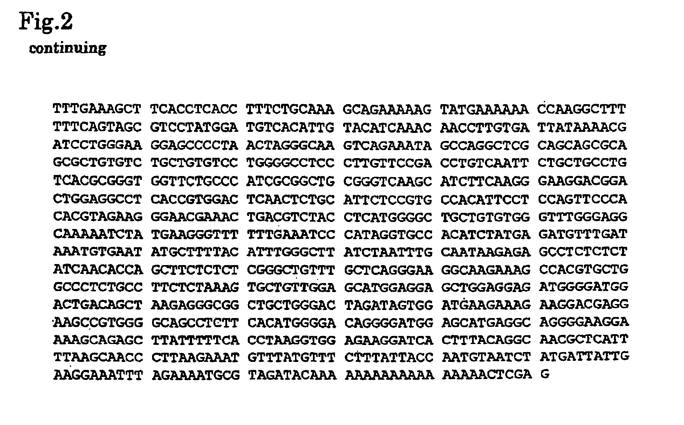

Fig. 1 shows the relationship between the nucleotide base sequence of a novel $PLA_1$ and the nucleotide base sequences obtained from an EST database. In the figure, ATG is an initiation codon, S, D, and H are active triads, C-C is a lid region, the broken line in an EST sequence is the region having a deletion in the EST base sequence. Fig. 2 shows the amino acid sequence of the novel $PLA_1$ and features of the sequence, as well the DNA sequence encoding the same. In the figure, a doubled underline shows a signal sequence, an underline shows a predicted site for addition of a sugar chain, the underline with arrow heads at both terminals shows a lipase consensus sequence and the lid region, S, D, and H surrounded with a square (shadowed) show active triads, and the square (opened) show an RGD sequence.

Fig. 3 shows a multiple alignment for comparison of amino acid sequences of the novel PLA$_1$ and lipases having homology with the novel PLA$_1$.

Fig. 4A is a schematic view of the structure of the lipase family and Fig. 4B shows the phylogenic tree of PLA$_1$/the lipase family.

Figs. 5A-5B showdetection of the novel PLA$_1$ recombinantly expressed in insect cells, by Western blotting. Fig 5A is a schematic view of a construct expressed, and Fig 5B shows the result of the Westernblotting.

Figs. 6A-6B show the results of purification of the novel PLA$_1$ by using a heparin column. Fig. 6A shows the results of fractionation using the heparin column and Fig. 6B shows the novel PLA$_1$ detected by Western blotting.

Figs. 7A-7C show the distribution of mRNA of the novel PLA$_1$ (Fig. 7A) and EDG7 (Fig. 7B) in various tissues. Fig. 7C shows expression of glyceraldehyde-3-phosphate dehydrogenase (G3PDH), which is a constitutively expressing gene used as an internal standard probe.

Fig. 8 shows expression of the novel PLA$_1$ protein in ovarian cancer cells and human platelets.

Fig. 9 shows a bioassay system using a cell in which the novel PLA$_1$ has been expressed, and an LPA receptor EDG7-expressing cell, in which Fura2 has been taken-up for examination of the action of the novel PLA$_1$.

Figs. 10A-10F show that Sf9 which expresses the novel PLA$_1$, has an increased intracellular Ca$^{2+}$ concentration compared to Sf9 cells which expresse the LPA receptor EDG7.

Figs. 11A-11F show that PLD is involved in LPA production mediated by the novel PLA$_1$.

**The Best Mode for Carrying Out the Invention**

**Novel PLA$_1$**

For the novel PLA$_1$ provided by the invention, a cDNA thereof was obtained from a cDNA library and encodes a novel amino acid sequence. The presence of the novel PLA$_1$ according to the invention was confirmed in human lung, kidney, pancreas, prostate, testis, ovary, and colon by the Northern blotting method. The properties of the novel PLA$_1$ according to the invention are as follows:

such produces LPA by acting on phospholipids, particularly phosphatidic acid (PA); has a high specific activity when using PA as a substrate; and has a consensus sequence and a catalyst triad, which are conserved among the lipase family, and an amino acid sequence which may be the lid. On the other hand, its homology with members of the known PLA$_1$ group is less than about 40%.

**Polypeptide or peptide**

The amino acid sequence of the novel PLA$_1$ of the invention is shown in SEQ ID NO:1. In addition, the polypeptide or the peptide of the invention is selected from the polypeptides or the peptides, which contain at least a portion of the polypeptide of SEQ ID NO:1. The polypeptide or the peptide of the invention has amino acid sequence homology of about 40% or higher, preferably about 70% or higher, more preferably about 80% or higher, further preferably about 90% or higher, particularly preferably about 95% or higher with the polypeptideof SEQ ID NO:1. The polypeptide or the peptide having such homology can be selected based on its ability to decompose phospholipids, particularly phosphatidic acid, and/or phosphatidic acid substrate specificity. The above-described decomposing activity can be measured using known methods such as using an radioisotope (RI) -labeled substrate, a fluorescent substrate, or a coloring substrate or the method described in the Examples herein (J. Biochem. 103: 442 - 447, 1988), (J. Biochem. 117: 1280 - 1287, 1995), (J. Biochem. 101: 53 - 61, 1987), (J. Biol. Chem. 235: 2595 - 2599, 1960), and (J. Biol. Chem. 272: 2192 - 2198, 1997).

Techniques for determining the amino acid sequence homology are known *per se* and are exemplified, for example, by a method for directly determining the amino acid sequence and a method for determining the nucleotide base sequence of the cDNA followed by deducing the amino acid sequence encoded thereby.

The polypeptide or the peptide of the invention includes a polypeptide or the peptide comprising a portion of the sequence of the polypeptide of SEQ ID NO:1. Such can be used, for example, as a reagent, a standard substance, or an immunogen. As a minimal unit thereof the polypeptide or the peptide preferably consists of an amino acid sequence of 8 or more consecutive amino acids, preferably 10 or more consecutive amino acids, more preferably 12 or more consecutive amino acids, further preferably 15 or more consecutive amino acids, and is preferably identifiable immunologically. These peptides can be used as the reagent or the standard substance, or, as describedherein, as an antigen for preparing an antibody specific to the novel PLA$_1$ independently or in combination with a carrier (for example, keyhole limpet hemocyanin or egg white albumin, or the like.) Moreover, polypeptides prepared by combining other species of proteins or other substances are included within the scope of the invention.

In addition, on the basis of such specified polypeptides or peptides, by using the presence of phospholipid decomposing activity, particularly phosphatidic acid decomposing activity, and/or the presence of phosphatidic acid substrate specificity as an index, a polypeptide or peptide is provided consisting of the amino acid sequence having variations or mutations such as a deletion, substitution, addition, and insertion of 1 or more, for example, 1 to 100, preferable 1 to 30, more preferably 1 to 20, further preferably 1 to 10, or particularly preferably 1 or several amino acids. The means for obtaining such a deletion, substitution, addition, and insertion is known *per se* and, for example, can be carried out, independently or in a proper combination, by the site specific mutagenesis method, gene homology recombination method, primer extension method, or polymerase chain reaction amplification method (PCR) and other methods described in, for example, "Molecular Cloning: a Laboratory Manual, 2nd ed. (ed. by Sambrook et al., published by Cold Spring Harbor Laboratory, 1989", "Lab Manual: Genetic Engineering (ed. by Muramatsu Masami. Published by Maruzen, 1988)", and "PCR Technology: Principle and Application of DNA Amplification. Edited by H. E. Ehrlich. Published by Stockton Press, 1989", or, methods modified from these methods, for example, by employing Ulmer's technique (Science 219: 666. 1983).

In making the mutants described above, one should consider maintaining the basic properties (physical properties, activity, immunological activity, or the like) of the protein, i.e., mutual substitution is easily supposed between, for example, homologous amino acids such as polar amino acids, non polar amino acids, hydrophobic amino acids, hydrophilic amino acids, positively charged amino acids, negatively charged amino acids, aromatic amino acids, or the like. As described later, the consensus sequence and the lid region of the lipase family are important for expression and regulation of the activity. The region comprising the same, particularly the consensus sequence comprising the catalyst triad, should be preferably maintained in the primary sequence and/or protein structure so as to maintain $PLA_1$ activity, particularly $PA-PLA_1$ activity. In addition, a polypeptide or the peptide of the invention is included within the scope of the invention, regardless of the presence and absence of the sugar chain. However, the sugar chain may influence the activity and therefore, at least one glycosylation site should be preferablymaintained.

In the invention, a polypeptide or the minimal unit thereof (region or domain) is provided, which has $PLA_1$ activity similar to that of the polypeptide having the amino acid sequence ofSEQ ID NO:1. In addition, the polypeptide having a modified activity level or modified substrate specificity is provided. Such are useful, for example, as a $PLA_1$ activity-like substance or a $PLA_1$ antagonistic substance for screening a substance regulating the $PLA_1$ activity. Further, a homologous gene product of an animal species other than human is included within the scope of the invention.

Moreover, in order to assist in detection and purification of the polypeptide or the like according to the invention or to add other functions, it is possible to add other proteins such as peptides, e.g., alkali phosphatase, β-galactosidase, an IgG such as an immunoglobulin Fc fragment, or FLAG-tag or the like to an N terminal side or a C terminal side directly, or indirectly by using a genetic engineering method through a linker peptide or the like. A polypeptide or the like bound to these other substances is included within the scope of the invention.

## Polynucleotide

In one aspect, the polynucleotide and the complementary chain thereof according to the invention mean a polynucleotide, which encodes the amino acid sequence of the polypeptide or the peptide according to the invention, for example, the amino acid sequence ofSEQ ID NO:1, and the complementary chain of the polynucleotide. These provide gene information useful for production of the above-described novel $PLA_1$, for example, or, can be used as a reagent or a standard for the nucleic acid. In SEQ ID NO:2, which shows a preferable polynucleotide, the regionfrom A (adenine) at nucleotide base number 89 to G (guanine) at nucleotide base number 1441 is the presumed coding region. On the other hand, atg to gaa encoding the region from M (met) at amino acid number 1 to E (Glu) at amino acid number 19 is presumed to encode a signal sequence. In addition, the presence of polymorphism between human individuals has been found in the sequences of the novel $PLA_1$ according to the invention. As an example, G (guanine) at nucleotide base number 1088 of SEQ ID NO:2 was substituted by T (thymine). As a result, D (Asp) at amino acid number 334 was substituted by Y (Tyr). In another example, A (adenine) at nucleotide base number 1204 of SEQ ID NO:2 was substituted by G (guanine) and in this example, it was determined that no amino acid substitution has occurred.

In another aspect, the invention provides the nucleotide encoding the amino acid sequence of the polypeptide or the peptide according to the invention, for example, the amino acid sequence ofSEQ ID NO:1, preferably the polynucleotide shown by the nucleotide base sequence of SEQ ID NO: 1, or a polynucleotide which hybridizes under stringent conditions to a region corresponding to the complementary chain thereof. The conditions of hybridization which can be employed, include, for example, those described in "Molecular Cloning: a Laboratory Manual, 2nd ed. (ed. by Sambrook et al., published by Cold Spring Harbor Laboratory, 1989".

These polynucleotides may not always be a complementary sequence, if they hybridize with the objective polynucleotide, particularly the polynucleotide shown by SEQ ID NO: 2 or complementary chain thereof. For example, the homology to the nucleotide base sequence of SEQ ID NO:2 or the complementary chain thereof is at least about 40%, for example, about 70% or higher, preferably about 80% or higher, more preferably about 90% or higher, further preferably about 95% or higher. The polynucleotide according to the invention includes nucleotides, polynucleotides or oligonucleotides which consist of "10 or more, preferably 15 or more, or more preferably 20 or morenucleotides" which correspond to a region of the nucleotide base sequence, and also includes complementary chains thereof.

These polynucleotides are, in the production of the polypeptide or the like of the invention, useful as a probe or a primer for detection of the nucleic acid encoding the novel $PLA_1$, e.g., the gene thereof, or of an mRNA thereof, and also as an antisense oligonucleotide to regulate gene expression. In this sense, the polynucleotide and the oligonucleotide of the invention may include not only a translated region, but also those corresponding to untranslated regions. For example, in order to specifically inhibit expression of the novel $PLA_1$ by an antisense oligonucleotide, a nucleotide base sequence of a region, which is other than the consensus sequence region conserved in the lipase family, inherent to the novel $PLA_1$ can be used. On the other hand, a conserved sequence may be used to suppress simultaneously expression of a plurality of lipases including the novel $PLA_1$. Herein, the nucleotide base sequence encoding the novel $PLA_1$ or the polypeptide having similar activity can be determined by, for example, confirming an expressed protein using a publicly known protein expression system and, then, selection is carried out using the physiological activity thereof, particularly, the phosphatidic acid decomposing activity as an index. In the case where a cell-free protein expression system is used, for example, the technique used may be based on a ribosome derived from a wheat germ, a rabbit reticular cell, or the like (Nature 179: 160 - 161, 1957).

**Transformant**

The peptide and the polypeptide consisting of the novel $PLA_1$ according to the invention and a derivative thereof can be obtained by gene recombination techniques, other than the cell-free protein expression system as described above, by using a host known *per se,* for example, Escherichia coli, yeast, Bacillus subtilis, insect cells, animal cells, and the like. In the specific example of the invention, insect cells were used, however, the invention is not restricted to this example (JP P2129487 B and JP P2644447 B: Method for producing recombinant baculovirus expression vector and synthesis of polypeptide). In addition, $PLA_1$ encoded by $PLA_1$ gene according to the invention is a glycoprotein and, therefore, use of a host such as animal cells is preferable, as such can add a sugar chain to the polypeptide or the peptide.

For transformation, means known *per se* are employed and, for example, transformation of the host cells is conducted by using a plasmid, a chromosome, a virus, and the like as a replicon. A more preferable system is, in consideration of the stability of the gene, a method involving integration in the chromosome. However, conveniently, an autonomous replication system is used with an extranuclear gene. The vector is chosen in accordance with the species of the host selected and contains the a gene sequence for expression and a gene sequence for replication and regulation, as structural elements. A combination is determined in accordance with the prokaryotic cell and eukaryotic cell selected, and by using a publicly known method *per se*. A promoter, a ribosome-binding site, a terminator, a signal sequence, an enhancer, and the like can be combined each other. In the specific example of the invention, a baculovirus system was used. Of course, the invention is not restricted to this example.

The transformant is cultured using conditions optimal and known *per se* for each host. The culture may be conducted using an enzyme activity, particularly phosphatidic acid decomposing activity, of the peptide and polypeptide consisting of the novel $PLA_1$ expressed and produced and the derivative thereof, as an index. Also, the production may be carried out by using a subculture or a batch culture and assigning "an amount of the transformant contained in a culture medium" as an index.

**Collecting novel $PLA_1$ and derivative thereof**

The peptide and the polypeptide consisting of the novel $PLA_1$ and the derivative thereof can be purified and collected from the culture medium by assigning the phosphatidic acid decomposing activity as an index and by combining molecular sieve, ion exchange column chromatography, affinity chromatography, and the like, or by means of ammonium sulfate fractionation, alcohol, and the like based on a difference in solubility. Preferably, on the basis of information about the amino acid sequence, an antibody against the amino acid sequence is prepared and then, the peptide and polypeptide are collected by a method of specific adsorbing - collecting using a polyclonal antibody or a monoclonal antibody. Conveniently, affinity chromatography using heparin can be used.

**Antibody**

The antibody is prepared by selecting an antigenic determinant of the peptide and the polypeptide consisting of the novel $PLA_1$ of the invention and the derivative thereof. The antigen may be the novel $PLA_1$ or a fragment thereof and is composed of at least 8, preferably at least 10, more preferably at least 12, and further preferably 15 or more amino acids. For preparation of the antibody specific to the novel $PLA_1$, it is preferable to use a region comprising a sequence inherent to the novel $PLA_1$ other than the region of the consensus sequence of the lipase family. This amino acid sequence is not necessarily homologousto SEQ ID NO:1, and a site exposed to outside of the protein structure is preferable. If the site exposed is a discontinuous site, the amino acid sequence is effectively continuous in the exposed site. The antibody is not specially restricted as long as it binds or recognizes immunologically the peptide and the polypeptide consisting of the novel $PLA_1$ and the derivative thereof. The presence or absence of this binding or recognition is determined by a publicly known antigen-antibody binding reaction.

The antibody is produced by using independently the peptide or the polypeptide, which consists of the novel $PLA_1$ of the invention and the derivative thereof, or by binding it to a carrier, and conducting immune induction, such as "humoral response and/or cellular response and the like", against an animal in the presence or absence of an adjuvant. The carrier is, unless itself causes a harmful action against the host, not specially restricted, and for example, includes cellulose, polymerized amino acids, albumin, and the like. As the animal to be immunized, a mouse, a rat, a rabbit, a goat, a horse, and the like are preferably used. The polyclonal antibody is obtained from serum by an antibody collection method known *per se.* A preferable means is immuno-affinity chromatography.

For production of the monoclonal antibody, an antibody producing cell (for example, the cell derived from a pancreas or a lymph node) is collected from the animal subjected to immunization as described above and a hybridoma is prepared by fusion with a permanent reproductive cell (for example, a myeloma cell line such P3X63Ag8 line) known *per se*. Th hybridoma is cloned, followed by selection for a hybridoma producing an antibody which specifically recognizes $PLA_1$ of the invention, and then the antibody is collected from the hybridoma culture supernatant.

The polyclonal antibody or the monoclonal antibody capable of suppressing the $PLA_1$ activity can be bound directly to the novel $PLA_1$ according to the invention to regulate the activity thereof and can regulate the system for producing LPA from phospholipids, particularly PA. Therefore, it is useful for therapeutic treatment and/or prevention of various malignant diseases related to LPA.

**Methods for identifying and screening some compounds**

"The peptide or the polypeptide consisting of the novel $PLA_1$ prepared in this way and the derivative thereof", "the polynucleotide encoding them and complementary chains thereof", "the cell transformed on the basis of information about these amino acid sequence and the nucleotide base sequence", "a protein synthesis system using them", and "the antibody which recognizes immunologically the peptide or the polypeptide consisting of the novel $PLA_1$ and the derivative thereof", independently or in combination of a plurality of them, provide an effective means for identifying and for screening for a substance or a agent regulating the activity of "the peptide and the polypeptide consisting of the novel $PLA_1$ and the derivative thereof" or the polynucleotide, e.g., as an inhibitor or activator. For example, selection of an antagonist in accordance with a drug design on the basis of the protein structure of the peptide or the polypeptide, selection of an expression-regulating agent on a gene level using the protein synthesis system, selection of an antibody-recognizing substance using the antibody, and the like can be used in a medicinal drug-screening system known *per se.* Herein, "regulation/-ing" as described above includes inhibition, antagonism, activation, activity promotion, activity endowment, and the like.

On the other hand, "the peptide or the polypeptide, which consists of the novel $PLA_1$ of the invention and the derivative thereof, or the polynucleotide or the transformant of the present invention", allows identification of "a compound which is able to activate or inhibit the activity of the peptide or the polypeptide consisting of the novel $PLA_1$ of the invention and the derivative thereof" or "a compound which is able to inhibit or promote expression of the polynucleotide according of the invention", by selecting " conditions capable of giving rise to interaction between the compound as a candidate for screening and the peptide or the polypeptide or the like", by employing a system using a signal (marker) detectable for the presence or absence of this interaction", and by detecting the "presence or absence of this signal (marker) or a change of a signal amount" thereof. The system using a signal (marker) includes a system of measuring the activity, such as the activity of decomposing a substrate such as PA, of the polypeptide of the invention or a system of measuring the amount of expressed amount of polynucleotide . Specifically, it will be exemplified in the Examples herein. For identification, a publicly known method may be applied.

By using the transformant, in which the polypeptide consisting of the novel $PLA_1$ of the invention and the derivative thereof has been expressed, and another transformant, which expresses a receptor of lysophosphatidic acid produced by action of "the polypeptide consisting of the novel $PLA_1$ expressed in the transformant or the derivative thereof" to phosphatidic acid, and contacting the above described polypeptide or transformants with the compound to be screened, and by detecting the presence or absence of a signal (marker) generated by interaction of the compound with the transformant, or a change thereof, under conditions where the compound and the above described polypeptide or these transformants are capable of interacting, the compound, which inhibits or activates the activity or a physiological action of "the polypeptide consisting of the novel $PLA_1$, the derivative thereof, or the polynucleotide of the invention", can be identified. The above described transformant includes, for example, a combination of Sf9 cells, in which the polypeptide consisting of the novel $PLA_1$ of the invention and the derivative thereof has been expressed, with Sf9 cells, in which the LPA receptor-EDG7 has been expressed, and is not restricted to this example. On the other hand, as the signal for detection of the action of the polypeptide consisting of the novel $PLA_1$ according to the invention and the derivative thereof, for example, it is sufficient to detect intracellular calcium whose content increases by combining LPA with an LPA receptor-EDG7-expressed cell. For detection of intracellular calcium, a measuring method known *per se* using Fura2 or the like can be applied. By comparing with a reaction in a control system, in which the polypeptide of the invention or the like has been replaced by another substance (for example, a polypeptide or the like,) which is homologous to lipase, or LPA, the specificity of the action of the compound can be confirmed. In addition, each transformant may be replaced by a cell line, in which expression of a corresponding gene has been confirmed.

**Compound and medicinal composition**

The compound identified in such a way can be used as a candidate compound of an inhibitor, antagonist, activating agent, promotor, or activator, which are related to the peptide and the polypeptide consisting of the novel $PLA_1$ and the derivative thereof. Further, the compound can be used as a candidate compound for an expression inhibitor, an expression antagonist, an expression activating agent, an expression accelerator, or an expression activator against the novel $PLA_1$ and the derivative thereof at the gene level. Prevention and/or therapy of various malignant symptoms derived from LPA can be expected with the same.

The candidate compound selected in this way may be prepared as a medicinal drug by choosing one taking into consideration a balance of biological usefulness and toxicity. The peptide or the polypeptide consisting of the novel $PLA_1$ of the invention and the derivative thereof, the polynucleotide encoding them and complementary chains thereof, the vector comprising the base sequences the same, and the antibody being able to recognize immunologically the peptide or the polypeptide consisting of the novel $PLA_1$ and the derivative thereof, themselves can be used as disease diagnostic means, such as a diagnostic marker or a reagent and medicinal drug means such as a remedy and the like using functions of inhibiting, antagonizing, activating, accelerating expression, activity, or action of the novel $PLA_1$. In preparing a medicinal drug, it is sufficient to introduce medicinal drug-preparing means known *per se* in accordance with each object, such as the peptide or the polypeptide, a protein, the polynucleotide, the antibody, and the like.

The medicinal composition as described above may be produced by using the peptide or the polypeptide consisting of the novel $PLA_1$ and the derivative thereof of the invention, the polynucleotide, the vector, the transformant, the antibody, and the compound of the invention as described above. The medicinal composition as described above is useful for therapeutic treatment of diseases related to $PLA_1$, particularly the novel $PLA_1$.

As the diagnostic means, it is useful to diagnose diseases related to the expression or the activity of the peptide or the polypeptide consisting of the novel $PLA_1$ of the invention and the derivative thereof. Diagnosis is, for example, carried out by determining the amount of an existing corresponding nucleic acid by using the interaction and reactivity to the nucleic acid sequence encoding the peptide, and/or determining a distribution of the peptide in a subject, and/or determining the presence of the peptide and the amount present or the amount of activity in a sample derived from thesubject, and the like. In other words, a test is conducted using the novel $PLA_1$ as the diagnostic marker. For a method for measurement, it is sufficient to use the antigen-antibody reaction system, an enzyme reaction system, PCR reaction system, and the like, which are known *per se.* Moreover, as described above, the presence of polymorphisms in accordance with individuals and thus, detecting a single nucleotide polymorphism (SNP) by a publicly known method is also useful as the diagnostic means.

**Example**

The invention will be specifically described as follows with reference to examples; however, the present invention is not restricted to the following examples.

**Isolation of the gene**

By using the amino acid sequence of rat phospholipase $A_1$ (PS-$PLA_1$) (J. Biol. Chem. 272, (4) : 2192-2198, 1997), which specifically dehydrates phosphatidyl serine, as a probe, a homology search (tblastn search) was carried out using a dbEST (database of Expressed Sequence Tags). As a result, two EST sequences having accession numbers AA149791 and AA102322, were found to have relatively higher homology scores among unknown nucleic acid sequences.

Next, a homology search (tblastn search) was carried out on dbEST using the nucleic acid sequence of accession number AA102322 as a probe.

As a result, it was found that there is a nucleotide base sequence region in which the sequence of accession number AA367368 is identical to a sequence of accession number AA149791 (Fig. 1). When these sequences were aligned in the identical region, it was found that alignment is possible in the order of AA149791 (sequence comprising a methionine residue thought to be a translation initiation codon) , AA102322 (sequence identical to AA149791), and AA367368 (sequence identical to AA102322 over a long range and comprising a catalyst triad) . Next, these sequences were aligned and features were analyzed. Based upon comparison with PS-PLA$_1$, the amino acid residue of an active triad, and a loop structure region called the lid (B. B. A. 1376: 417 - 432, 1998), (Biochemistry 32: 4702 - 4707, 1993) (Protein, Nucleic Acid, and Enzyme 44: 1038 - 1042, 1999) around an active pocket in a protein structure, of which latter two portions are specific to lipases, was identified. From features of the sequence, it was presumed to be a novel phospholipase A$_1$.

**Cloning of the novel sequence**

In order to clone the cDNA comprising the novel PLA$_1$ gene sequence, which was predicted from the above-described analysis, a clone comprising a partial cDNA sequence (accession number AA149791), which was derived from a human colon and believed to comprise the initiation methionine codon, was obtained from the American Type Culture Collection (ATCC).

By confirming the nucleic acid sequence of the clone, it was believed to be possible to identify a sequence comprising the full-length cDNA. In order to confirm on the basis of the sequence that such was not an artifact, oligonucleotides having the nucleotide base sequence of 5'-TGCGAAGTAAATCATTCTTGTGAA-3' (a forward primer sequence) (SEQ ID NO:3) and the nucleotide base sequence of 5' -TGTGACATCCATAGGACGCTACTG-3' (a reverse primer sequence) (SEQ ID NO:4) was prepared as PCR primers and RT-PCR was conducted by using RNA's (Clontech) derived from a human colon, lung, and kidney. The nucleotide base sequence of a gene fragment (about 1.5 kbp) that was thus amplified was determined after plasmid pBlueScript II SK (Stratagene) was used as the vector for cloning, and cloning such into a multi-cloning site thereof, EcoRI/XhoI. The sequencing was carried out using the primer of the multi-cloning site of pBlueScript II SK and using 4 restriction sites of EcoRI, PstI, HindIII, and XhoI. Next, on the basis of this sequence, a primer oligomer was designed and the nucleotide base sequence, which was believed to be the novel PLA$_1$ (SEQ ID NO:2) was finally confirmed by applying the primer walking technique (Fig. 2). In addition, the sequence was also confirmed by direct sequencing of an RT-PCR product. Through these steps, polymorphism in 2 sites of the nucleotide base sequence was found.

Escherichia coli containing the plasmid comprising the nucleotide base sequence was deposited under accession number FERM P-17428 at the Research Institute of Bioscience and Human Technology on June 22, 1999. This deposit was transferred to an international deposit on June 15, 2000 (FERM BP-7188).

The cDNA of SEQ ID NO:2 contains an open reading frame comprising 1353 bases, which can encode a protein consisting of 451 amino acid residues (SEQ ID NO:4), and has a region thought to be a signal sequence in an N terminal region. The sequence has four N-{P}-[ST]-{P} sites in [N (Asn) 50-C (Cys) 53, N (Asn) 58-A (Ala) 61, N (Asn) 66-K (Lys) 69, and N (Asn) 357-E (Glu) 360] as an asparagine glycosylation site motiff, contains one site (R (Arg) 344 - D (Asp) 346) of a RDG sequence known as a cell-associated region motiff, and also has 13 cysteine residues.

**Homology with existing protein**

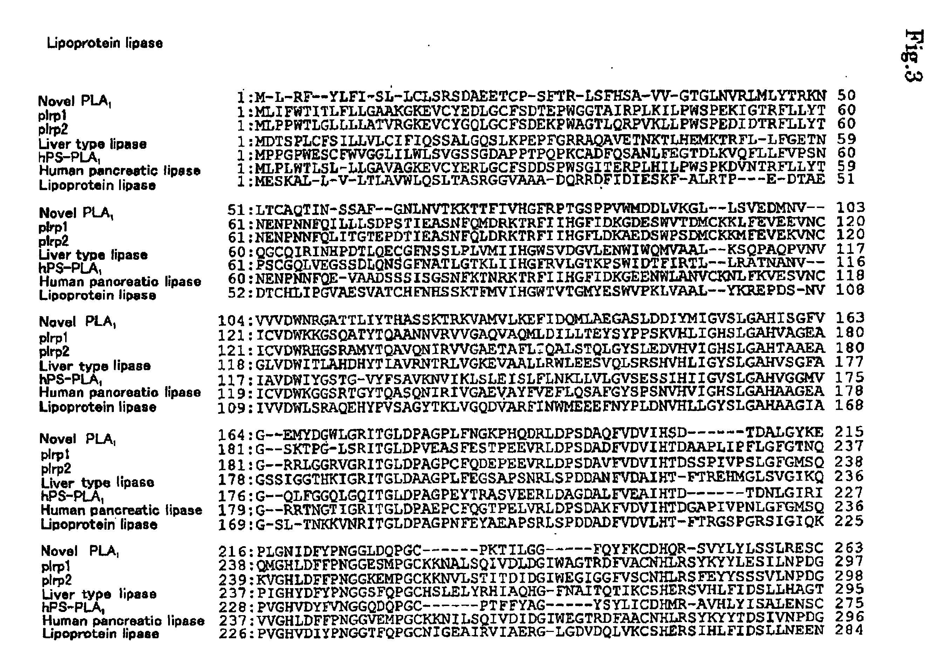

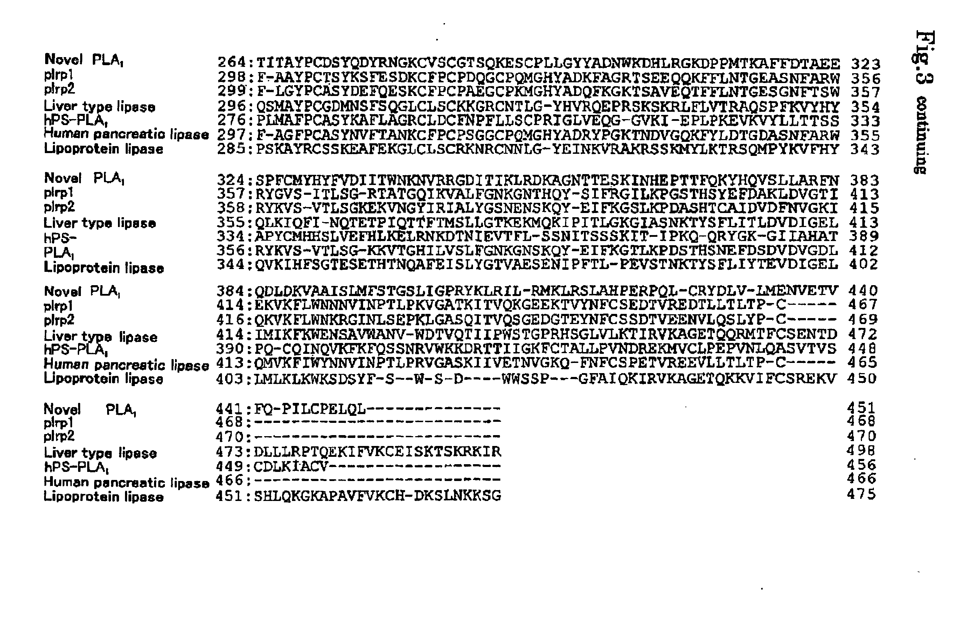

By using the amino acid sequence predicted upon translation of the nucleotide base sequence, a homology search was conducted using tblastn in an existing database (GenBank) . As shown in Fig. 3, the novel lipase (novel PLA$_1$) (colon lipase) of the invention showed significant homology with human PS-PLA$_1$ (hPS-PLA$_1$), pancreas type lipase (human pancreatic lipase), liver type lipase (hepatic lipase), lipoprotein lipase, plrp1 (pancreatic lipase relatedprotein 1), andplrp2 (pancreatic lipase related protein 2). On the other hand, high homology was found in vitellogenin, which is thought to have a protein-structural region highly homologous with lipase. In the protein as described above, excluding vitellogenin among these known protein sequences having high homology, it was confirmed that all of the amino acid residues, (S (Ser) 154, D (Asp) 178, H (His) 248), which are predicted as the enzyme active triad are conserved, and hence, a multiple alignment table was prepared from the sequences by using a GENETYX Multiple Alignment module (Software Development, K. K).

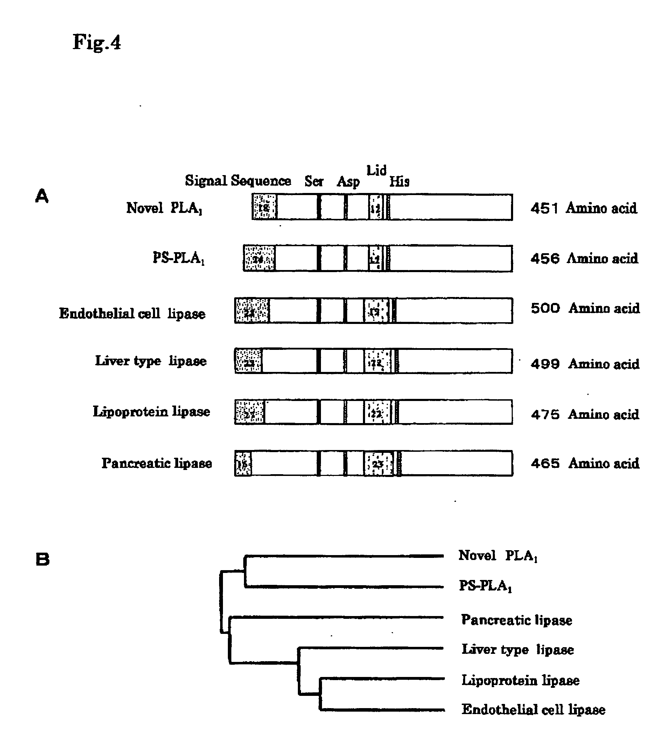

As a result, in the amino acid sequence of SEQ ID NO:1, as shown in Fig. 2, it was found that there is a consensus sequence GXSXG [G (Gly) 152 - G (Gly) 156], ITGLD(I (Ile) 174 - D (Asp)178), and CXH [C(Cys) 246 - H (His) 248] (X is an arbitrary amino acid), and that these sequences contain all of the amino acid residues believed to be the enzyme active triads. It was also found that 12 residues of loop structures [P (Pro) 234 - K (Lys) 245) called lids, are present around the pocket, which has the protein-structure of the active triad, and regulates the activity of lipase, and that this number of lids is equal to number of the residues of PS-PLA$_1$. As a rule, it has been known that a group of lipases other than PS-PLA$_1$ has a large number of amino acid residues with the lid structure, and the activity is expressed by binding a proteinaceous factor called colipase (B. B. A. 1376: 417 - 432, 1998), (Biochemistry 32: 4702 - 4707, 1993) and (Protein, Nucleic Acid, and Enzyme 44: 1038 - 1042, 1999). However, for PS-PLA$_1$ having a relatively short lid, the necessity of colipase has not been found. Therefore, it is predicted that the protein translated from the nucleotide base sequence expresses its activity through mechanisms similar to those of PS-PLA$_1$. Fig. 4A shows a comparison of the novel PLA$_1$ with other PLA$_1$ families in a schematic diagram. Next, by using GYNETYX Evolutional Tree (a tool for the UPGMA method) (Software Development, K. K.), an evolutional phylogenic tree of the sequence of the PLA$_1$ lipase family was predicted. As a result, it is believed that the novel sequence has a sequence evolutionarily closest to that of PS-PLA$_1$ (Fig. 4B). As described above, the protein produced by translation of the novel sequence is close to lipase group, and is a novel lipase particularly close to phospholipase.

**Expression of novel PLA$_1$**

A full-length cDNA was prepared by digesting the above-described pBlueScript II SK (-)with EcoRI/XhoI. The cDNA was incorporated in the multi-cloning site of plasmid pF$_{AST}$B$_{AC}$1 (Life Tech Oriental Corp.) using the EcoRI/XhoI restriction enzyme site. In order to attach FLAG - tag (Asp Tyr Lys Asp Asp Asp Asp Lys) (Biotechnology 6: 1205 - 1210, 1988) to the C terminal, the termination codon was removed and in its place, a synthetic oligonucleotide (primer 2) comprising a HindIII site at the terminal of the nucleic acid sequence encoding FLAG - tag (SEQ ID NO:6) was prepared, and also the oligonucleotide (primer 1) was prepared by introducing BamHI at the beginning of the initiation methionine (SEQ ID NO:5), and then PCR was conducted using the above-described pBlueScript II SK (-) as a template for amplification of the cDNA.

```
Primer 1: 5' - CGC GGA TCC ATG TTG AGA TTC TAC TTA TTC ATC - 3' (SEQ

ID NO:5)


Primer 2: 5' - CCG GAA TTC TTA CTT GTC ATC GTC GTC CTT GTA GTC CAA CTG

CAA CTC TGG GCA AAG AAT - 3' (SEQ ID NO:6)
```

After the cDNA was incorporated at the BamHI/EcoRI restriction enzyme site of the multi-cloning site in plasmid pF$_{AST}$B$_{AC}$1, the constructed pF$_{AST}$B$_{AC}$1 was transfected into Escherichia coli JM109 and a positive clone was chosen and, then, the positive clone was cultured to collect the plasmid. This plasmid was transfected into DH10BAC™ competent cell (Gibco BRL) to collect a recombined Bacmid. The bacmid obtained was transfected into Sf9 cells (derived from Spodoptera frugiperda pupa ovary tissue) together with Cell FECTIN™ (pF$_{AST}$B$_{AC}$1). As a result, a recombinant baculovirus was collected in the culture supernatant.

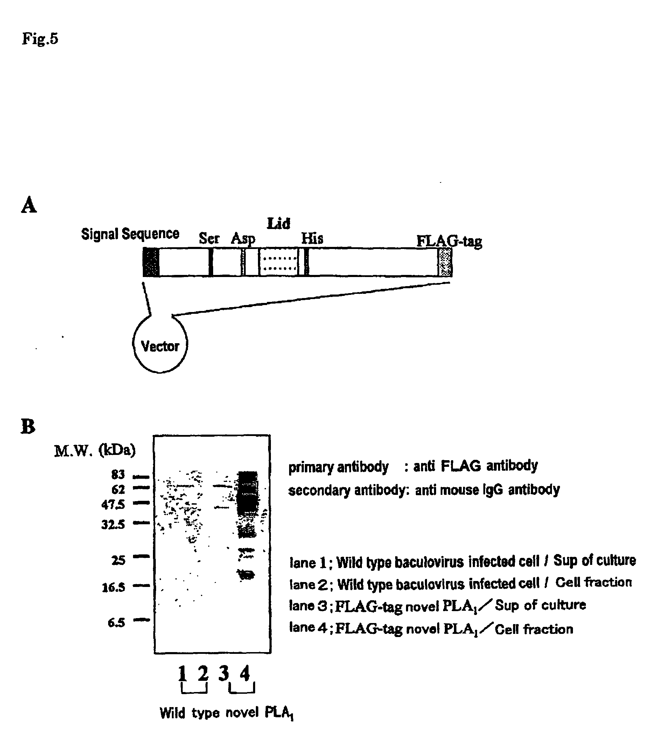

Next, in order to confirm expression of the protein, Sf9 insect cells were infected with the collected baculovirus and subsequently cultured at 27 °C for 96 h. The infected and cultured Sf9 cells were centrifuged to separate such into a cell fraction and a supernatant, and a protein extract from each was subjected to SDS-PAGE and Western blotting was conducted using an antibody against FLAG-tag. The novel PLA$_1$ was located in the supernatant of the culture in a small amount, and a large portion thereof was collected from the cell fraction. A plurality of bands was observed in front of and behind the about 50 kDa expected molecular weight of the novel PLA$_1$ (Fig 5B). These plural bands are believed to be caused by modification of the sugar chain of the novel PLA$_1$. The novel PLA$_1$ has a sequence similar to that of a signal peptide at the N terminal of the amino acid sequence, and is believed to be a cell-associated enzyme. Hereinafter, cell association means presence in a cell membrane or in a cell or presence associated with the cell membrane. By the technique described above, it was confirmed that the novel PLA$_1$ could be expressed in using insect cells or the like. On the other hand, for purification of the novel PLA$_1$ as described below, the purifying process was simplified by using the culture supernatant which contains a small amount of the novel PLA$_1$.

**Purification of novel PLA$_1$**

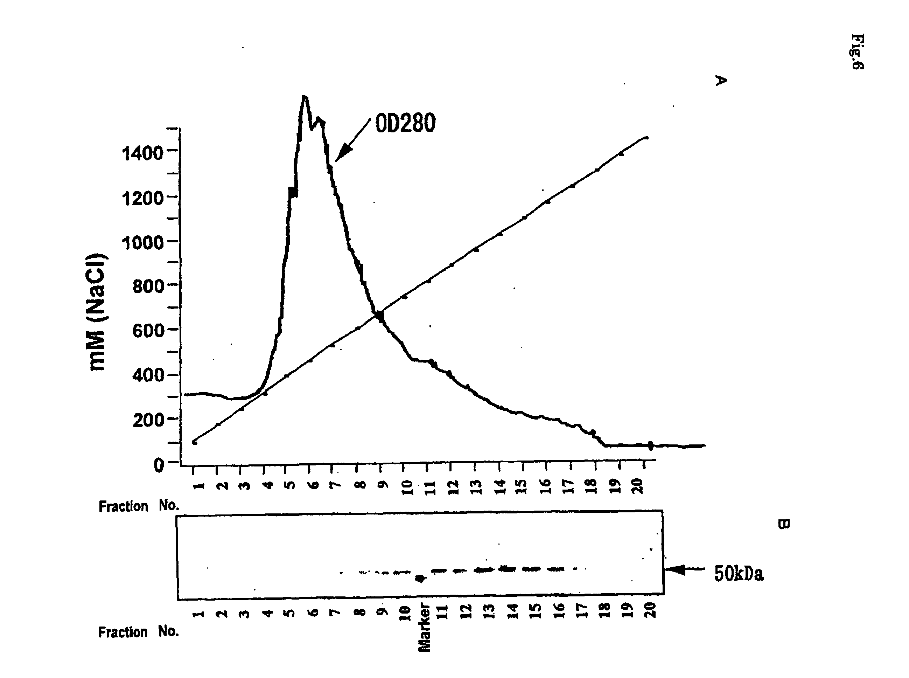

500 ml of the supernatant of the culture infected with the recombinant baculovirus (JP P2129487 B and JP P2644447 B: Method for producing recombinant baculovirus expression vector and synthesis of polypeptide) was collected, cell fragments were removed by centrifugal operation at 10000 X g, for 20 min, and at 4 °C, and debris was removed from the culture supernatant by filtering (Falcon, pore size 0.45 μ m). Using the FPLC system (Amersham-Pharmacia), the culture supernatant described above was applied to a heparin column (Hi-trap Heparin, Amersham-Pharmacia, 5 ml) for final concentration gradient elution with 100 mM to 1500 mM NaCl in the presence of 10 mM Tris-HCl (pH 7.4). Fractionation of an eluate was carried out every 2.5 ml and 20 fractions were separated in total. The novel $PLA_1$ was eluted at a relatively high concentration of NaCl, about 1 M, and showed high affinity to heparin (Fig. 6A). Next, a portion of individual fractions fractionated was subjected to SDS-PAGE and subsequent Western blotting using an anti-FLAG-tag antibody, and finally, it was confirmed that the novel $PLA_1$ having a molecualr weight of about 50 kDa was collected in fraction numbers from 10 to 16 (Fig. 6B).

Similarly, as a control experiment, the culture supernatant infected with wild-type baculovirus was applied to the heparin column as described above and concentration gradient NaCl elution fractions were obtained. However, in these fractions, a molecule detectable using the anti-FLAG-tag antibody was not found.

## Preparation of antibody

A peptide having a sequence of 18 amino acids (from amino acid number 434 (Met) to amino acid number 451 (Leu) ofSEQ ID NO:1) in the C terminal of the novel $PLA_1$ was bound to KLH (keyhole limpet hemocyanin) for use as an antigen and applied to the back of a foot pad of a rat (WYK line) together with Freund's complete adjuvant for immunization. A lymph node cell of the rat thus immunized was fused with a myeloma cell (PAI) of a mouse to yield fused cells and among them, an antibody-secreting cell was chosen by a conventional ELISA screen, cell fluorescence method, and Western blotting. An antibody against the novel $PLA_1$ was obtained by culturing a selected hybridoma cell .

## Confirming expressed tissue

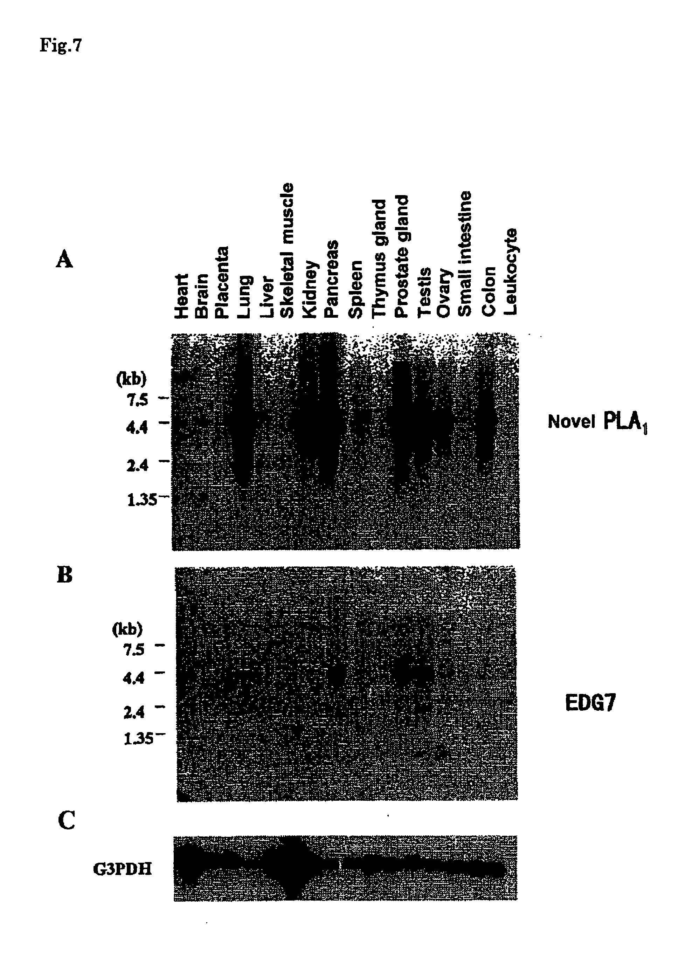

In order to examine the tissue in which the novel $PLA_1$ was expressed, Northern blotting was carried out using human normal tissue. A PCR fragment of about 0.7 kbp which was a cDNA fragment of the open reading frame, was used as the probe. In other words, the oligonucleotide having the nucleotide base sequence of CTGCGCACAAACCATCAACTCCTC (the forward primer sequence) (SEQ ID NO:7) and AGGGGACAGGACTCTTTTTGTGAC (the reverse primer sequence) (SEQ ID NO:8) was prepared as PCR primers, and PCR was conducted to prepare a $^{32}P$ labeled probe. Northern blotting was carried out using a Human Multiple Tissue Northern Blot (Clontech) according to the user's manual (PT1200-1, Clontech). As a result, in the normal tissue, expression of the mRNA was observed in the lung, kidney, pancreas, prostate, testes, ovary, and colon (Fig. 7A).

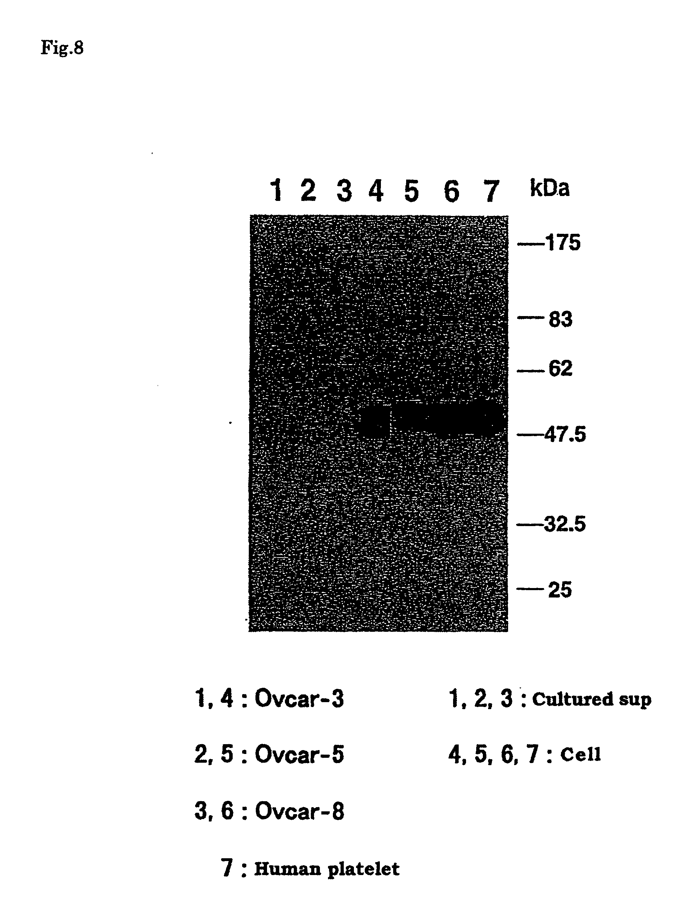

Further, in several human ovarian cancer cell lines (Ovcar-3, Ovcar-5, and Ovcar-8), it was found by Northern blotting that the mRNA of the novel $PLA_1$ was expressed. In addition, for the ovarian cancer cell lines as described above, Western blotting analysis was carried using the monoclonal antibody, as described above, against the novel $PLA_1$ ($sPA-PLA_1$) and, two bands of 55 kDa and 52 kDa were detected, and thus it was clear that the novel $PLA_1$ protein was expressed in these cell lines (Ovcar-3, Ovcar-5, and Ovcar-8) (Fig. 8). The bands of two different molecular weights represent differences in the sugar chain modification of the novel $PLA_1$. Almost all of the novel $PLA_1$ was collected from the cell fraction of the ovarian cancer cell lines and not obtained from the cell supernatant. Moreover, the novel $PLA_1$ was highly expressed in human platelets (Fig. 8). For the platelets, two bands were detected, however, the molecular weights thereof were somewhat lower than that observed in ovarian cancer cells. Intracellular localization of the novel $PLA_1$ in the ovarian cancer cell was examined by immunofluorescence using the novel $PLA_1$ monoclonal antibody described above.Specifically, these ovarian cancer cell lines (Ovcar-3, Ovcar-5, and Ovcar-8) were cultured in a Dulbecco's modified Eagle culture medium (DMEM) containing 5% fetal calf serum (FCS) in 5% $CO_2$ atmosphere. Cells propagated on a cover glass were fixed with ice-cooled methanol and blocked with 10% goat serum. The cells were then incubated together with the antibody against the novel $PLA_1$ or an anti-caveolin 1 antibody (Santa Cruz Biotech Corp.) and subsequently, further incubated together with rat or rabbit anti-Ig antigen (Alexa488/green or Alexa 594/red) as a second antibody, and finally, subjected to double staining using the antibody against the novel $PLA_1$ and the anti-caveolin 1 antibody. An intracellular region, where the novel $PLA_1$ or caveolin was present, was detected by a fluorescence microscope (Zeiss, Germany). As a result, it was found that the novel $PLA_1$ was localized in the same region as that of caveolin 1, which is known to be localized in a micro-domain of the cell surface. On the other hand, in the Ovcar-5 cells, the novel $PLA_1$ showed a spotted distribution.

## Test of substrate specificity

Using the above described novel $PLA_1$ purified from the Sf9 cells infected with the recombinant baculovirus, and phosphatidic acid (PA), whose fatty acid was radioisotope-labeled by [$^{14}C$], phosphatidyl serine (PS), phosphatidyl chorine (PC), or triacylglycerol (TG) as substrates, phospholipase activity and lipase activity were measured (J. Biol. Chem. 272: 2192 - 2198, 1997). For the phospholipase activities (PA), (PS) and (PC) 40 $\mu M$ of each substrate was incubated together with the enzyme in the presence of 100 mM Tris-HCl (pH 7.5), and 4 mM of $CaCl_2$ at 37 °C for 1 h, released radioisotope-labeled fatty acid was extracted by the Dole method, and finally, radioactivity was measured using a liquid scintillation counter. For lipase activity (TG), 40 µM of each substrate was incubated in the presence of 100 mM Tris-HCl (pH 7.5) and 4 mM $CaCl_2$ at 37 °C for 1 h together with the enzyme, released radioisotope-labeled fatty acid was extracted by the Dole method, and radioactivity was measured using a liquid scintillation counter. As a result, phospholipase activity cleaving PA and PS was detected for the novel $PLA_1$. For lipase activity cleaving TG and phospholipase activity cleaving PC or PE, a significant activity was not detected. Phospholipase activity cleaving PA was not detected in a fraction purified in the same way using Sf9 cell culture supernatant infected with wild-type baculovirus. From the above, it is believed that the novel $PLA_1$ hydrolyzes PA and is involved in a process of production of lysophosphatidic acid (LPA), which is a growth factor promoting differentiation and/or proliferation of cells.

**Comparison with PS-PLA$_1$**

Table 1 shows a comparison of properties of the novel $PLA_1$ of the invention with known human phosphatidyl serine-specific phospholipase $A_1$ (PS-PLA$_1$). The novel $PLA_1$ of the present invention, in comparison with PS-PLA$_1$, showed high affinity with heparin and almost all of the same was a glycoprotein with cell - binding activity.

Table 1

|  | Lid | Heparin Affinity | Organ Distribution | Intracellular Localization | Substrate Specificity |
|---|---|---|---|---|---|
| PS- PLA$_1$ (456a.a.) | 12 a.a. | Low | inducible | Secretory Cell-associated | PA LysoPS |
| Novel PLA$_1$ (451a.a.) | 12 a.a. | High | inducible | Cell-associated Secretory | PA PS |

**Relationship with LPA receptor**

The novel $PLA_1$ works on PA to hydrolyze and produce 2-acyl LPA. Therefore, it is believed that the novel $PLA_1$ produces LPA for supplying to the LPA receptor as a ligand. The LPA receptors known are EDG2, EDG4, and EDG 7. Among them, EDG 7 is a unique receptor showing a strong reactivity to LPA comprising an unsaturated fatty acid and reacting more strongly to 2-acyl LPA than 1-acyl LPA. Ligand specificity thereof is different from that of EDG2 and EDG4 (J. Biol. Chem. 274: 27776 - 27785, 1999). It is thought that signal transmission through EDG7 is mediated by a $PLA_1$ reaction and hence, expression of the novel $PLA_1$ and EDG7 in the living body was examined by Northern blotting. mRNAs of both of these substances were found to be expressed in the pancreas, prostate, and testes and showed relatively similar tissue distribution (Fig. 7A, B, and C). Further, as described above, in an ovarian cancer cell line and platelets showing expression of the novel $PLA_1$, the presence of PLA was also found.

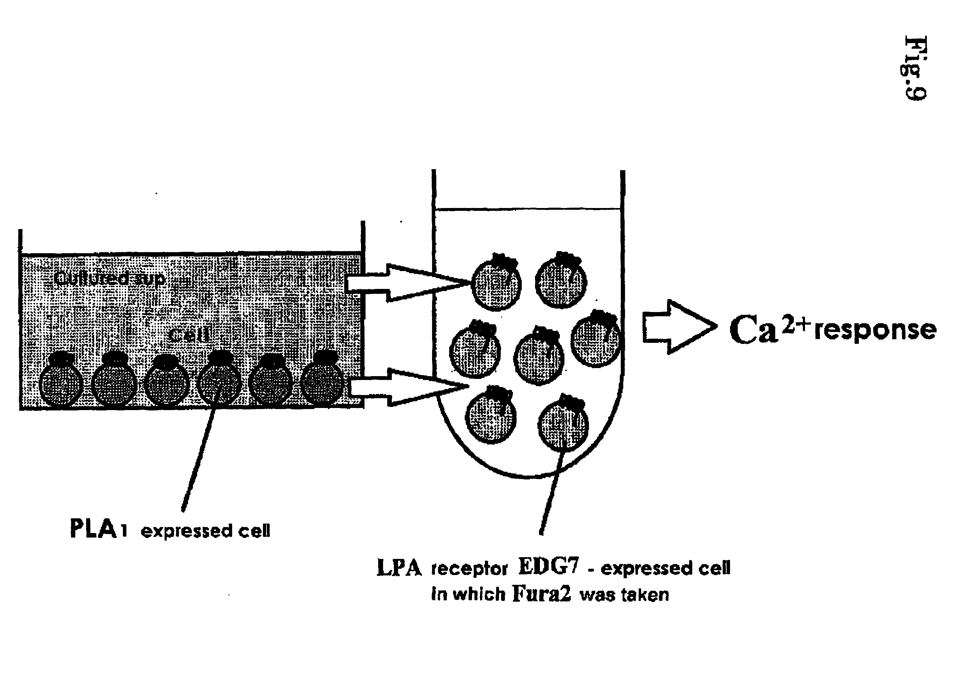

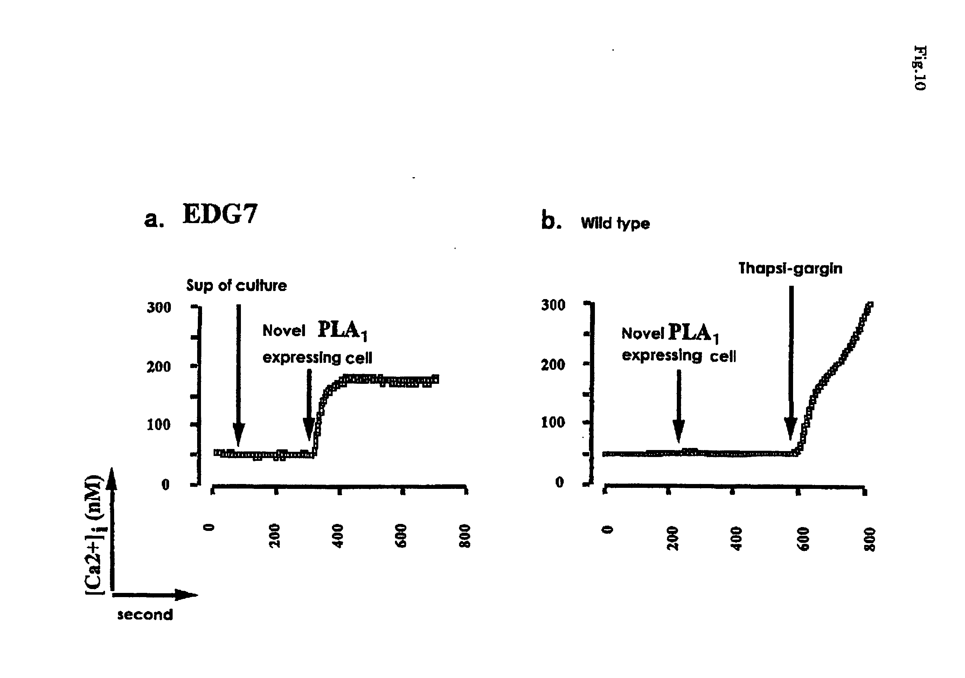

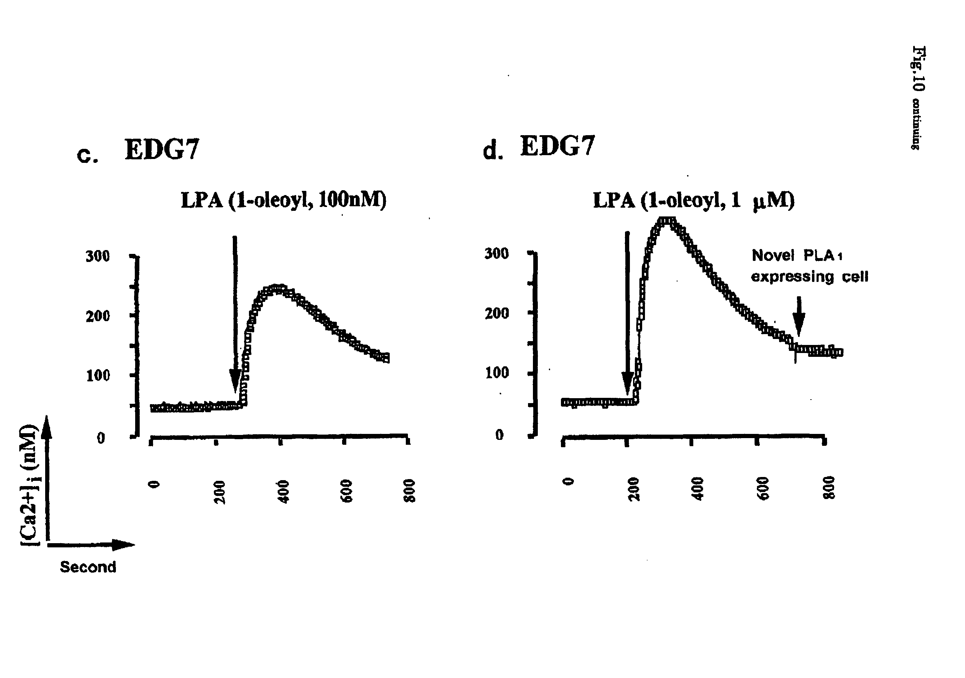

Next, the possibility that the novel $PLA_1$ hydrolyzes PA and produce LPA for supplying to EDG7 as the ligand was examined. First of all, the novel $PLA_1$, of the invention, partially purified was added to the cell expressed EGD7, however, a cellular response was not observed. Then, by using the bioassay system shown in Fig. 9, it was examined whether LPA can be produced by the novel $PLA_1$. As the bioassay system, an insect cell Sf9 lacking reactivity to LPA was used. The novel $PLA_1$ was expressed in Sf9 cells using the baculovirus system as described above (hereafter occasionally enzyme side). Meanwhile, EDG7 as the LPA receptor was expressed in the Sf9 cells using the baculovirus system according to the method in J. Biol. Chem. 274: 27776 - 27785, 1999 (hereafter occasionally receptor side.) In this system, if the novel $PLA_1$ is expressed enough to produce LPA, LPA binds to the cell expressing the LPA receptor and causes intracellular signal transmission, resulting in a rise in the intercellular concentration of calcium ions ($Ca^{2+}$). In other words, LPA production and the action of the novel $PLA_1$ *in vitro* can be examined by detecting a change in the $Ca^{2+}$ concentration. The change in the $Ca^{2+}$ concentration was measured by using the $Ca^{2+}$ fluorescence indicator Fura-2. First of all, the Sf9 cells expressing the LPA receptor were suspended in a nutrient liquid (10 mM $CaCl_2$, 60 mM KCl, 17 mM $MgCl_2$, 10 mM NaCl, 10 mM MES, 4 mM glucose, 110 mM sucrose, and 0.1% bovine serum albumin) at a concentration of $5 \times 10^5$ cells/ml and 2 µM Fura2-AM was added to the cells at 27 °C for 1 h. Then, the cells were washed twice with the nutrient liquid as described above, and then, suspended in the nutrient liquid at a concentration of $5 \times 10^5$ cells/ml. The Sf9 cells expressing the novel $LPA_1$ were suspended in the nutrient liquid at a concentration of $5 \times 10^5$ cells/50 µl. 1 ml of the LPA receptor-expressing cells were added to a cuvette, stirred by a micro-stirrer, and irradiated with 340 nm and 380 nm excitation light. Next, the fluorescence intensity and ratios thereof at 500 nm were measured using a CAF-110 type intracellular ion measurement instrument (JASCO Corporation.) 50 µl of the novel $LPA_1$-expressing cells were added thereto for measurement in the same way. Moreover, at the time of each measurement, one value, when all Fura 2 and extracellular $Ca^{2+}$'s were bound by addition of Triton-X100, and another value, when all $Ca^{2+}$'s were chelated by addition of EGTA to dissociate Fura2, was simultaneously measured. By using each measured value as described above; an intracellular calcium concentration was calculated from the following formula.

$$[Ca^{2+}] \ (nM) = 224 \ X \ "b"/"a" \ X \ ("F"-"Fmin")/("Fmax"-"F")$$

In the formula, 224 represents a dissociation constant of Fura 2, "a" represents the fluorescence intensity at 380 nm excitation light when all Fura 2 was bound to extracellular $Ca^{2+}$'s upon addition of Triton-X100, "b" represents the fluorescence intensity at 380 nm excitation light when Fura 2 was dissociated upon addition of EGTA to chelate all $Ca^{2+}$'s, "F" represents the ratio of the fluorescence intensity at 340 nmexcitation light/the fluorescence intensity at 380 nmexcitation light, "Fmax" represents "F" when all Fura 2's were bound to extracellular $Ca^{2+}$'s upon addition of Triton-X100, "Fmin" represents "F" when Fura 2 was dissociated upon addition of EGTA to chelate all $Ca^{2+}$'s.

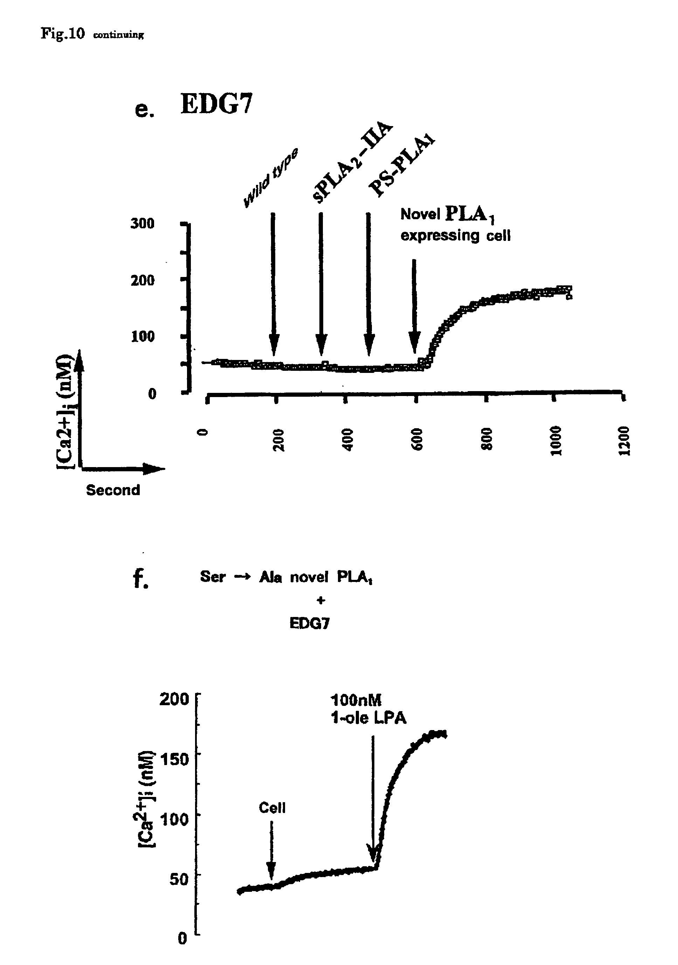

As a result, when the novel $PLA_1$-expressing cells were added to the EDG7-expressed SF9 cells, a rise in the intercellular $Ca^{2+}$ concentration was observed (Fig. 10a). This phenomenon was not observed, when the receptor side (Fig. 10b) or the enzyme side (Fig. 10c) was changed to the cells infected with wild-type baculovirus. That Fura 2 was taken-up at the receptor side cell was confirmed by using thapsi-gargin discharging $Ca^{2+}$ from an intracellular $Ca^{2+}$ store (Fig. 10b). On the other hand, it was found that a rise of the intercellular $Ca^{2+}$ concentration was, after the $Ca^{2+}$ concentration was temporarily raised by LPA (100 nM 1-oleoyl) (Fig. 10c), not observed, when the novel $PLA_1$-expressing cells were added and subjected to desensitization (Fig. 10d). This suggests that the observed rise of the $Ca^{2+}$ is mediated by EDG7. This intercellular $Ca^{2+}$ concentration of the EDG7-expressed cells cannot be induced by the cells, in which other phospholipase [$sPLA_2$-IIA (secretory phospholipase type IIA) or PS-$PLA_1$] was expressed (Fig. 10e). Therefore, this is a phenomenon specific to the novel $LPA_1$. Meanwhile, it was found that the culture supernatant of the novel $PLA_1$-expressing cells never induced any detectable $Ca^{2+}$ reaction (Fig. 10a) and the LPA produced was almost all associated with the cells. In addition, for cells expressing the novel $PLA_1$ mutant, in which a serine residue [amino acid sequence 154 of SEQ ID NO:1 (Ser)] in the enzyme - active center of the novel $PLA_1$ and conserved in almost all lipase/$PLA_1$ families, was substituted to an alanine residue, there was no induced rise in the $Ca^{2+}$ of the EDG7-expressed cells (Fig. 10f). From the above-described results, it was proven that the novel $PLA_1$ hydrolyzes endogenous intracellular PAas the substrate, andproduces LPA which acts on the cells expressing EDG7, the LPA receptor. Further, it was suggested that the LPA produced is present in the cell membrane. As described above, intracellular LPA production by the novel $PLA_1$ and mechanisms for transfer of LPA present in the cell membrane to EDG7, the LPA receptor, were confirmed herein. In addition, a tool for elucidating the physiological significance of the $PLA_1$ family and the action mechanisms thereof are provided hereby.

**Involvement of PLD in LPA production mediated by the novel $PLA_1$**

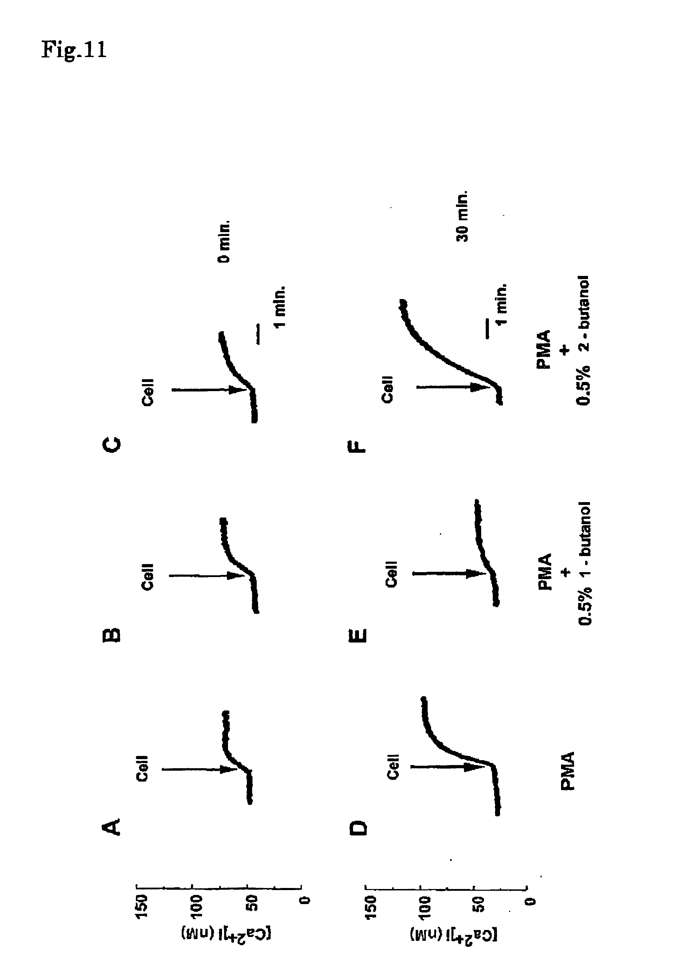

Phospholipase D (PLD), which converts a membrane phospholipid to PA is also involved in LPA production by ovarian cancer cells. In order to find out the role of PLD in LPA production by the novel $PLA_1$, PMA (phorbol 12-myr-istate 13-acetate) activating $PLD_1$ through protein kinase $C\alpha$ and a short-chain alcohol inhibiting all isoforms of PLD were used. The novel $PLA_1$-expressed cells and the EDG7-expressed cells, which were used, were identical to those as described above. First of all, the novel $PLA_1$-expressed cells were incubated in the presence of PMA to examine the capability of induction of calcium discharge from the EDG7-expressed cells. As shown in Figs 11A and D, treatment of the cells with 100 nM PMA for 30 min significantly accelerated the rise of the intercellular $Ca^{2+}$ concentration, which was caused by adding the novel $PLA_1$-expressed cells. Moreover, in the presence of 0.5% 1-butanol, which concentration allows for complete inhibition of the PLD activity, acceleration of the rise of the $Ca^{2+}$ concentration in the novel $PLA_1$-expressed cells by PMA treatment was completely inhibited (Fig. 11B and E). On the other hand, 0.5% 2-butanol did not affect the same (Fig. 11C and F). 2-butanol does not inhibit PLD and therefore, it can be said that LPA production by the novel $PLA_1$ depends on PLD in the Sf9 cells. A single use of PMA, 1-butanol, or 2-butanol did not have any effect on the intercellular $Ca^{2+}$ concentration. In addition, PMA treatment of the Sf9 cells, in which other phospholipase, PS-$PLA_1$, or $sPLA_2$-IIa were expressed, caused no induction of any detectable $Ca^{2+}$ reaction in the EDG7-expressed cells. In other words, the novel $PLA_1$ produces LPA (probably, 2-acyl-1-lysoPA) by cooperation with activation of PLD.

**Preparation of antisense oligo**

A phosphorothioate type oligonucleotide having a nucleotide sequence GAATAAGTAGAATCTCAACATATGG [a complementary chain corresponding to the region comprising the initiation codon from C (cytosine) of nucleotide base number 85 to C (cytosine) of nucleotide base number 109 of SEQ ID NO:2] was synthesized by Sawady Technology, K.K..

**Preparation of a full length antisense (complementary chain) expression vector**

A cDNA fragment was amplified by PCR using Bluescript SK-PA-$PLA_1$ (identical with that described above), which is the plasmid comprising the DNA encoding the novel $PLA_1$, as a template DNA, and digested by restriction enzymes BamHI and NotI. The obtained DNA fragment of about 1.5 kbp was ligated in the BamHI - NotI sites of vector pcDNA3 for expression to prepare a vector expressing the antisense chain (complementary chain) to the full-length sequence.

**Test of suppressing $PLA_1$ expression**

The antisense oligo and the full-length antisense expression vector, which were prepared as described above, were transfected into cell lines Ovcar-3, Ovcar-5, and Ovcar-8, which express the novel $PLA_1$, using a transfection reagent FuGene6 (Roche) according to a user's manual. As a control, vector pcDNA3 was used. After 72 hours, the cells were collected and conventional Western blotting was conducted. [WHAT ARE THE RESULTS OF THIS EXPERIMENT?]

**Industrial Applicability**

The present invention provides a novel $PLA_1$ belonging to the $PLA_1$ lipase family. The novel $PLA_1$, which is a cell-associated glycoprotein, has substrate specificity for phosphatidic acid (PA) and hydrolyzes PA to produce lysophosphatidic acid (LPA). In addition, the invention provides information regarding the physiological significance of the $PLA_1$ family and mechanisms for production of a ligand of the LPA receptor, on the basis of LPA production in a cell by a novel PA-specific lipase ($PLA_1$) and the presence of mechanisms for transfer of LPA from the cell to EDG7, the LPA receptor. A novel medicinal drug composition and a therapeutic means, as an application of these findings will be useful for clinical and basic medical fields related to lipase.

**Claims**

1. A polypeptide selected from the group consisting of:

    (a) a polypeptide consisting of the amino acid sequence of SEQ ID NO:1;
    (b) a polypeptide comprising the polypeptide of (a);
    (c) a polypeptide having at least 70% amino acid sequence homology with the polypeptide of (a) and having phosphatidic acid decomposing activity; and
    (d) a polypeptide having variation of one or more amino acids in the amino acid sequence of SEQ ID NO:1, such as a deletion, substitution, addition, and insertion in the amino acid sequence, and having the phosphatidic acid decomposing activity.

2. A peptide comprising at least about 8 consecutive amino acids of the amino acid sequenceof SEQ ID NO:1.

3. A polynucleotide encoding the polypeptide of claim 1 or the peptide of claim 2, or a complementary chain thereof.

4. A polynucleotide which hybridizes to the polynucleotide of claim 3 under stringent conditions, or a complementary chain thereof.

5. A polynucleotide consisting of at least about 15 consecutive nucleotide bases of the polynucleotide of SEQ ID NO: 2 or a complementary chain thereof.

6. A recombinant vector comprising any one of the polynucleotides of claims 3 to 5.

7. A transformant transformed by the recombinant vector of claim 6.

8. A method for producing the polypeptide of claim 1 or of the peptide of claim 2, comprising culturing the transformant of claim 7.

9. An antibody which specifically binds the polypeptide of claim 1 or the peptide of claim 2.

10. The antibody of claim 9, wherein said antibody is able to suppress phosphatidic acid decomposing activity.

11. A method for identifying a compound which interacts with the polypeptide of claim 1 so as to inhibit or activate the activity of said polypeptide, and/or a compound which interacts with the polynucleotide of claim 3 or claim 4 so as to inhibit or accelerate expression thereof, wherein the method comprises a step using at least one member selected from the group consisting of the polypeptide of claim 1 or the peptide of claim 2, the polynucleotides of claims 3 to 5, the vector of claim 6, the transformant of claim 7, and the antibodies of claim 9 or claim 10.

12. A method for identifying a compound which interacts with the polypeptide of claim 1 so as to inhibit or activate the activityof said polypeptide, and/or a compound which interacts with the polynucleotide of claim 3 or claim 4 so as to inhibit or accelerate expression thereof, comprising the steps of:

    evaluating the interaction of the compound by contacting the polypeptide or the polynucleotide with the compound to be screened under conditions allowing an interaction of the compound with the polypeptide or the polynucleotide (such interaction is related to a second component capable of providing a detectable signal responding to the interaction of the compound with the polypeptide or the polynucleotide); and
    detecting the presence or absence or a change of a signal generated by the interaction of the compound with the polypeptide or the polynucleotide to determine whether the compound interacts with the polypeptide or the polynucleotide and activates or inhibits the activities thereof.

13. A method for identifying a compound which inhibits or activates "the activity or a physiological action" of "the polypeptide of claim 1" or "the polynucleotide of claim 3 or claim 4", comprising the steps of:

    evaluating, using the transformant of claim 7 and another transformant expressing a receptor for lysophosphatidic acid produced upon action of "the polypeptide of claim 1 expressed in said transformant" to phosphatidic acid, the interaction with the compound by contacting the transformants with the compound to be screened under conditions allowing for interaction of the compound with the transformants (such interaction is related to the second component capable of providing a detectable signal responding to the interaction of the compound with the transformant); and

detecting the presence or absence or a change of a signal generated by the interaction of the compound with the transformant to determine whether the compound is able to activate or inhibit "the activity or the physiological action" of "the polypeptide or the polynucleotide".

14. A compound identified by the methods of claims 11 to 13.

15. A compound which interacts with the polypeptide of claim 1 so as to inhibit or activate the activity thereof, or a compound which interacts with "the polynucleotide of claim 3 or claim 4" so as to inhibit or accelerate the expression thereof.

16. A medicinal composition comprising at least one member selected from the group consisting of the polypeptide of claim 1 or the peptide of claim 2, the polynucleotides of any one of claims 3 to 5, the vector of claim 6, the transformant of claim 7, the antibodies of claim 9 or claim 10, and the compounds of claim 14 or claim 15.

17. A method for diagnosing a disease related to "expression or the activity" of the polypeptide of claim 1 in a subject, comprising analyzing (a) a nucleic acid sequence encoding the polypeptide and/or (b) the polypeptide contained in a sample obtained from the subject, as a marker.

18. A method for therapeutic treatment comprising administering the medicinal composition of claim 16 to a subject afflicted with a disease related to phospholipase $A_1$.

19. A method for producing the medicinal composition of claim 16.

Fig. 1

EP 1 298 205 A1

## A relation between a base sequence of a novel PLA₁ and the base sequence obtained from an EST database

PA-PLA₁

ATG ... S D H ... C-C (Lid)

0 500 1000 1500 2000 2400

AA149791
AA102322
AA367368

# Fig.2

```
                                                                    GAATTCGG
CACGAGAAAA  TCCCACAGTG  GAAACTCTTA  AGCCTCTGCG  AAGTAAATCA  TTCTTGTGAA
TGTGACACAC  GATCTCTCCA  GTTTCCATAT  GTTGAGATTC  TACTTATTCA  TCAGTTTGTT
                                    M    L   R   F    Y   L   F   L    S   L   L
GTGCTTGTCA  AGATCAGACG  CAGAAGAAAC  ATGTCCTTCA  TTCACCAGGC  TGAGCTTTCA
 C   L   S   R   S   D   A   E   T        C   P   S    F   T   R   L    S   F   H
CAGTGCAGTG  GTTGGTACGG  GACTAAATGT  GAGGCTGATG  CTCTACACAA  GGAAAAACCT
 S   A   V   V   G   T   G.   L   N   V    R   L   M.   L   Y   T   R    K   N   L
GACCTGCGCA  CAAACCATCA  ACTCCTCAGC  TTTTGGGAAC  TTGAATGTGA  CCAAGAAAAC
 T   C   A   Q   T   I   N   S   S   A.   F   G   N    L   N   V   T    K   K   T
CACCTTCATT  GTCCATGGAT  TCAGGCCAAC  AGGCTCCCCT  CCTGTTTGGA  TGGATGACTT
 T   F   I   V   H   G   F    R   P   T    G   S   P    P   V   W   M    D   D   L
AGTAAAGGGT  TTGCTCTCTG  TTGAAGACAT  GAACGTAGTT  GTTGTTGATT  GGAATCGAGG
 V   K   G   L   L   S   V    E   D   M    N   V   V    V   V   D   W    N   R   G
AGCTACAACT  TTAATATATA  CCCATGCCTC  TAGTAAGACC  AGAAAAGTAG  CCATGGTCTT
 A   T   T   L   I   Y   T   .H   A   S    S   K   T    R   K   V   A.   M   V   L
GAAGGAATTT  ATTGACCAGA  TGTTGGCAGA  AGGAGCTTCT  CTTGATGACA  TTTACATGAT
 K   E   F   I   D   Q   M    L   A   E    G   A   S    L   D   D   I    Y   M   I
CGGAGTAAGT  CTAGGAGCCC  ACATATCTGG  GTTTGTTGGA  GAGATGTACG  ATGGATGGCT
 G   V   S   L   G   A   H    I   S   G    F   V   G    E   M   Y   D    G   W   L
GGGGAGAATT  ACAGGCCTCG  ACCCTGCAGG  CCCTTTATTC  AACGGGAAAC  CTCACCAAGA
 G   R   I   T   G   L   S    P   A   G    P   L   F    N   G   K   P    H   Q   D
CAGATTAGAT  CCCAGTGATG  CGCAGTTTGT  TGATGTCATC  CATTCCGACA  CTGATGCACT
 R   L   D   P   S   D   A    Q   F   V    D   V   I    H   S   D   T    D   A   L
GGGCTACAAG  GAGCCATTAG  GAAACATAGA  CTTCTACCGA  AATGGAGGAT  TGGATCAACC
 G   Y   K   E   P   L   G    N   I   D    F   Y   P    N   G   G   L    D   Q   P
TGGCTGCCCC  AAAACAATAT  TGGGAGGATT  TCAGTATTTT  AAATGTGACC  ACCAGAGGTC
 G   C   P   K   T   I   L    G   G   F    Q   Y   F    K   C   D   S    Q   R   S
TGTATACCTG  TACCTGTCTT  CCCTGAGAGA  GAGCTGCACC  ATCACTGCGT  ATCCCTGTGA
 V   Y   L   Y   L   S   S    L   R   E    S   C   T    I   T   A   Y    P   C   D
CTCCTACCAG  GATTATAGGA  ATGGCAAGTG  TGTCAGCTGC  GGCACGTCAC  AAAAAGAGTC
 S   Y   Q   D   Y   R   N    G   K   C    V   S   C    G   T   S   Q    K   E   S
CTGTCCCCTT  CTGGGCTATT  ATGCTGATAA  TTGGAAAGAC  CATCTAAGGG  GGAAAGATCC
 C   P   L   L   G   Y   Y    A   D   N    W   K   D    H   L   R   G    K   D   P
TCCAATGACG  AAGGCATTCT  TTGACACAGC  TGAGGAGAGC  CCATTCTGCA  TGTATCATTA
 P   M   T  .K   A   F   F    D   T   A    E   E   S    P   F   C   M    Y   H   Y
CTTTGTGGAT  ATTATAACAT  GGAACAAGAA  TGTAAGAAGA  GGGGACATTA  CCATCAAATT
 F   V   D   I   I   T   W    N   K   N    V   R   R    G   D   I   T    I   K   L
GAGAGACAAA  GCTGGAAACA  CCACAGAATC  CAAAATCAAT  CATGAACCCA  CCACATTTCA
 R   D   K   A   G   N   T    T   E   S    K   I   N    H   E   P   T    T   F   Q
GAAATATCAC  CAAGTGAGTC  TACTTGCAAG  ATTTAATCAA  GATCTGGATA  AAGTGGCTGC
 K   Y   H   Q   V   S   L    L   A   R    F   N   Q    D   L   D   K    V   A  A
AATTTCCTTG  ATGTTCTCTA  CAGGATCTCT  AATAGGCCCA  AGGTACAAGC  TCAGGATTCT
 I   S   L   M   F   S   T    G   S   L    I   G   P    R   Y   K   L    R   I   L
CCGAATGAAG  TTAAGGTCCC  TTGCCCATCC  GGAGAGGCCT  CAGCTGTGTC  GGTATGATCT
 R   M   K   L   R   S   L    A   H   P    E   R   P    Q   L   C   R    Y   D   L
TGTCCTGATG  GAAAACGTTG  AAACAGTCTT  CCAACCTATT  CTTTGCCCAG  AGTTGCAGTT
 V   L   M   E   N   V   E    T   V   F    Q   P   I    L   C   P   E    L   Q   L
GTAACTGTTG  CCAGGACACA  TGGCCATAAA  TAATAGAAAG  AAAGCTACAA  CCACAGGCTG
 *
```

# Fig.2

continuing

```
TTTGAAAGCT TCACCTCACC TTTCTGCAAA GCAGAAAAAG TATGAAAAAA CCAAGGCTTT
TTTCAGTAGC GTCCTATGGA TGTCACATTG TACATCAAAC AACCTTGTGA TTATAAAACG
ATCCTGGGAA GGAGCCCCTA ACTAGGGCAA GTCAGAAATA GCCAGGCTCG CAGCAGCGCA
GCGCTGTGTC TGCTGTGTCC TGGGGCCTCC CTTGTTCCGA CCTGTCAATT CTGCTGCCTG
TCACGCGGGT GGTTCTGCCC ATCGCGGCTG CGGGTCAAGC ATCTTCAAGG GAAGGACGGA
CTGGAGGCCT CACCGTGGAC TCAACTCTGC ATTCTCCGTG CCACATTCCT CCAGTTCCCA
CACGTAGAAG GGAACGAAAC TGACGTCTAC CTCATGGGGC TGCTGTGTGG GTTTGGGAGG
CAAAAATCTA TGAAGGGTTT TTTGAAATCC CATAGGTGCC ACATCTATGA GATGTTTGAT
AAATGTGAAT ATGCTTTTAC ATTTGGGCTT ATCTAATTTG CAATAAGAGA GCCTCTCTCT
ATCAACACCA GCTTCTCTCT CGGGCTGTTT GCTCAGGGAA GGCAAGAAAG CCACGTGCTG
GCCCTCTGCC TTCTCTAAAG TGCTGTTGGA GCATGGAGGA GCTGGAGGAG ATGGGGATGG
ACTGACAGCT AAGAGGGCGG CTGCTGGGAC TAGATAGTGG ATGAAGAAAG AAGGACGAGG
AAGCCGTGGG GCAGCCTCTT CACATGGGGA CAGGGGATGG AGCATGAGGC AGGGGAAGGA
AAAGCAGAGC TTATTTTTCA CCTAAGGTGG AGAAGGATCA CTTTACAGGC AACGCTCATT
TTAAGCAACC CTTAAGAAAT GTTTATGTTT CTTTATTACC AATGTAATCT ATGATTATTG
AAGGAAATTT AGAAAATGCG TAGATACAAA AAAAAAAAAA AAAAACTCGA G
```

Fig.3

EP 1 298 205 A1

Lipoprotein lipase

```
Novel PLA₁            1:M-L-RF--YLFI-SL-LCLSRSDAEETCP-SFTR-LSFHSA-VV-GTGLNVRLMLYTRKN  50
plrp1                 1:MLIFWTITLFLLGAAKGKEVCYEDLGCFSDTEPWGGTAIRPLKILPWSPEKIGTRFLLYT  60
plrp2                 1:MLPPWTLGLLLLATVRGKEVCYGQLGCFSDEKPWAGTLQRPVKLLPWSPEDIDTRFLLYT  60
Liver type lipase     1:MDTSPLCFSILLVLCIFIQSSALGQSLKPEPFGRRAQAVETNKTLHEMKTRFL-LFGETN  59
hPS-PLA₁              1:MPPGPWESCFWVGGLILWLSVGSSGDAPPTPQPKCADFQSANLFEGTDLKVQFLLFVPSN  60
Human pancreatic lipase 1:MLPLWTLSL-LLGAVAGKEVCYERLGCFSDDSPWSGITERPLHILPWSPKDVNTRFLLYT  59
Lipoprotein lipase    1:MESKAL-L-V-LTLAVWLQSLTASRGGVAAA-DQRRDFIDIESKF-ALRTP---E-DTAE  51

Novel PLA₁           51:LTCAQTIN-SSAF--GNLNVTKKTTFIVHGFRPTGSPPVWMDDLVKGL---LSVEDMNV-- 103
plrp1               61:NENPNNFQILLLSDPSTIEASNFQMDRKTRFIIHGFIDKGDESWVTDMCKKLFEVEEVNC 120
plrp2               61:NENPNNFQLITGTEPDTIEASNFQLDRKTRFIIHGFLDKAEDSWPSDMCKKMFEVEKVNC 120
Liver type lipase   60:QGCQIRINHPDTLQECGFNSSLPLVMIIHGWSVDGVLENWIWQMVAAL--KSQPAQPVNV 117
hPS-PLA₁           61:PSCGQLVEGSSDLQNSGFNATLGTKLIIHGFRVLGTKPSWIDTFIRTL--LRATNANV-- 116
Human pancreatic lipase 60:NENPNNFQE-VAADSSSISGSNFKTNRKTRFIIHGFIDKGEENWLANVCKNLFKVESVNC 118
Lipoprotein lipase  52:DTCHLIPGVAESVATCHFNHSSKTFMVIHGWTVTGMYESWVPKLVAAL--YKREPDS-NV 108

Novel PLA₁          104:VVVDWNRGATTLIYTHASSKTRKVAMVLKEFIDQMLAEGASLDDIYMIGVSLGAHISGFV 163
plrp1              121:ICVDWKKGSQATYTQAANNVRVVGAQVAQMLDILLTEYSYPPSKVHLIGHSLGAHVAGEA 180
plrp2              121:ICVDWRHGSRAMYTQAVQNIRVVGAETAFLTQALSTQLGYSLEDVHVIGHSLGAHTAAEA 180
Liver type lipase  118:GLVDWITLAHDHYTIAVRNTRLVGKEVAALLRWLEESVQLSRSHVHLIGYSLGAHVSGFA 177
hPS-PLA₁          117:IAVDWIYGSTG-VYFSAVKNVIKLSLEISLFLNKLLVLGVSESSIHIIGVSLGAHVGGMV 175
Human pancreatic lipase 119:ICVDWKGGSRTGYTQASQNIRIVGAEVAYFVEFLQSAFGYSPSNVHVIGHSLGAHAAGEA 178
Lipoprotein lipase 109:IVVDWLSRAQEHYPVSAGYTKLVGQDVARFINWMEEEFNYPLDNVHLLGYSLGAHAAGIA 168

Novel PLA₁         164:G--EMYDGWLGRITGLDPAGPLFNGKPHQDRLDPSDAQFVDVIHSD------TDALGYKE 215
plrp1             181:G--SKTPG-LSRITGLDPVEASFESTPEEVRLDPSDADFVDVIHTDAAPLIPFLGFGTNQ 237
plrp2             181:G--RRLGGRVGRITGLDPAGPCFQDEPEEVRLDPSDAVFVDVIHTDSSPIVPSLGFGMSQ 238
Liver type lipase 178:GSSIGGTHKIGRITGLDAAGPLFEGSAPSNRLSPDDANFVDAIHT-FTREHMGLSVGIKQ 236
hPS-PLA₁         176:G--QLFGGQLGQITGLDPAGPEYTRASVEERLDAGDALFVEAIHTD------TDNLGIRI 227
Human pancreatic lipase 179:G--RRTNGTIGRITGLDPAEPCFQGTPELVRLDPSDAKFVDVIHTDGAPIVPNLGFGMSQ 236
Lipoprotein lipase 169:G-SL-TNKKVNRITGLDPAGPNFEYAEAPSRLSPDDADFVDVLHT-FTRGSPGRSIGIQK 225

Novel PLA₁         216:PLGNIDFYPNGGLDQPGC------PKTILGG-----FQYFKCDHQR-SVYLYLSSLRESC 263
plrp1             238:QMGHLDFFPNGGESMPGCKKNALSQIVDLDGIWAGTRDFVACNHLRSYKYYLESILNPDG 297
plrp2             239:KVGHLDFFPNGGKEMPGCKKNVLSTITDIDGIWEGIGGFVSCNHLRSFEYYSSSVLNPDG 298
Liver type lipase 237:PIGHYDFYPNGGSFQPGCHSLELYRHIAQHG-FNAITQTIKCSHERSVHLFIDSLLHAGT 295
hPS-PLA₁         228:PVGHVDYFVNGGQDQPGC------PTFFYAG-----YSYLICDHMR-AVHLYISALENSC 275
Human pancreatic lipase 237:VVGHLDFFPNGGVEMPGCKKNILSQIVDIDGIWEGTRDFAACNHLRSYKYYTDSIVNPDG 296
Lipoprotein lipase 226:PVGHVDIYPNGGTFQPGCNIGEAIRVIAERG-LGDVDQLVKCSHERSIHLFIDSLLNEEN 284
```

Fig.3 continuing

EP 1 298 205 A1

```
Novel PLA₁              264:TITAYPCDSYQDYRNGKCVSCGTSQKESCPLLGYYADNWKDHLRGKDPPMTKAFFDTAEE 323
plrp1                   298:F-AAYPCTSYKSFESDKCFPCPDQGCPQMGHYADKFAGRTSEEQQKFFLNTGEASNFARW 356
plrp2                   299:F-LGYPCASYDEFQESKCFPCPAEGCPKMGHYADQFKGKTSAVEQTFFLNTGESGNFTSW 357
Liver type lipase       296:QSMAYPCGDMNSFSQGLCLSCKKGRCNTLG-YHVRQEPRSKSKRLFLVTRAQSPFKVYHY 354
hPS-PLA₁                276:PLMAFPCASYKAFLAGRCLDCFNPFLLSCPRIGLVEQG-GVKI-EPLPKEVKVYLLTTSS 333
Human pancreatic lipase 297:F-AGFPCASYNVFTANKCFPCPSGGCPQMGHYADRYPGKTNDVGQKFYLDTGDASNFARW 355
Lipoprotein lipase      285:PSKAYRCSSKEAFEKGLCLSCRKNRCNNLG-YEINKVRAKRSSKMYLKTRSQMPYKVFHY 343

Novel PLA₁              324:SPFCMYHYFVDIITWNKNVRRGDITIKLRDKAGNTTESKINHEPTTFQKYHQVSLLARFN 383
plrp1                   357:RYGVS-ITLSG-RTATGQIKVALFGNKGNTHQY-SIFRGILKPGSTHSYEFDAKLDVGTI 413
plrp2                   358:RYKVS-VTLSGKEKVNGYIRIALYGSNENSKQY-EIFKGSLKPDASHTCAIDVDFNVGKI 415
Liver type lipase       355:QLKIQFI-NQTETPIQTTFTMSLLGTKEKMQKIPITLGKGIASNKTYSFLITLDVDIGEL 413
hPS-                    334:APYCMHHSLVEFHLKELRNKDTNIEVTFL-SSNITSSSKIT-IPKQ-QRYGK-GIIAHAT 389
PLA₁                    356:RYKVS-VTLSG-KKVTGHILVSLFGNKGNSKQY-EIFKGTLKPDSTHSNEFDSDVDVGDL 412
Lipoprotein lipase      344:QVKIHFSGTESETHTNQAFEISLYGTVAESENIPFTL-PEVSTNKTYSFLIYTEVDIGEL 402

Novel PLA₁              384:QDLDKVAAISLMFSTGSLIGPRYKLRIL-RMKLRSLAHPERPQL-CRYDLV-LMENVETV 440
plrp1                   414:EKVKFLWNNNVINPTLPKVGATKITVQKGEEKTVYNFCSEDTVREDTLLTLTP-C----- 467
plrp2                   416:QKVKFLWNKRGINLSEPKLGASQITVQSGEDGTEYNFCSSDTVEENVLQSLYP-C----- 469
Liver type lipase       414:IMIKFKWENSAVWANV-WDTVQTIIPWSTGPRHSGLVLKTIRVKAGETQQRMTFCSENTD 472
hPS-PLA₁                390:PQ-CQINQVKFKFQSSNRVWKKDRTTTIIGKFCTALLPVNDREKMVCLPEPVNLQASVTVS 448
Human pancreatic lipase 413:QMVKFIWYNNVINPTLPRVGASKIIVETNVGKQ-FNFCSPETVREEVLLTLTP-C----- 465
Lipoprotein lipase      403:LMLKLKWKSDSYF-S--W-S-D----WWSSP---GFAIQKIRVKAGETQKKVIFCSREKV 450

Novel     PLA₁         441:FQ-PILCPELQL----------------                                   451
plrp1                  468:----------------------------                                   468
plrp2                  470:----------------------------                                   470
Liver type lipase      473:DLLLRPTQEKIFVKCEISKTSKRKIR                                     498
hPS-PLA₁               449:CDLKIACV--------------------                                   456
Human pancreatic lipase 466:----------------------------                                   466
Lipoprotein lipase     451:SHLQKGKAPAVFVKCH-DKSLNKKSG                                     475
```

# Fig.4

A

| | Signal Sequence | Ser | Asp | Lid His | |
|---|---|---|---|---|---|
| Novel PLA₁ | | | | | 451 Amino acid |
| PS-PLA₁ | | | | | 456 Amino acid |
| Endothelial cell lipase | | | | | 500 Amino acid |
| Liver type lipase | | | | | 499 Amino acid |
| Lipoprotein lipase | | | | | 475 Amino acid |
| Pancreatic lipase | | | | | 465 Amino acid |

B

Novel PLA₁
PS-PLA₁
Pancreatic lipase
Liver type lipase
Lipoprotein lipase
Endothelial cell lipase

Fig.5

**A**

Signal Sequence — Ser — Asp — **Lid** — His — FLAG-tag

Vector

**B**

M.W. (kDa)

83
62
47.5
32.5
25
16.5
6.5

primary antibody : anti FLAG antibody
secondary antibody: anti mouse IgG antibody

lane 1; Wild type baculovirus infected cell / Sup of culture
lane 2; Wild type baculovirus infected cell / Cell fraction
lane 3; FLAG-tag novel $PLA_1$／Sup of culture
lane 4; FLAG-tag novel $PLA_1$／Cell fraction

1 2 3 4

Wild type novel $PLA_1$

Fig.6

Fig.7

A — Novel PLA₁

B — EDG7

C — G3PDH

Fig.8

1, 4 : Ovcar-3          1, 2, 3 : Cultured sup

2, 5 : Ovcar-5          4, 5, 6, 7 : Cell

3, 6 : Ovcar-8

7 : Human platelet

Fig.9

PLA1 expressed cell

LPA receptor EDG7 - expressed cell
in which Fura2 was taken

Cultured sup

Cell

$Ca^{2+}$ response

Fig.10

Fig.10 *continuing*

EP 1 298 205 A1

c. **EDG7**

d. **EDG7**

Fig.10 continuing

### e. EDG7

### f.    Ser → Ala novel PLA₁
+
EDG7

Fig.11

## INTERNATIONAL SEARCH REPORT

International application No.

PCT/JP00/04441

**A. CLASSIFICATION OF SUBJECT MATTER**
Int.Cl⁷  C12N9/16, C12N15/55, C07K16/40, A61K38/43

According to International Patent Classification (IPC) or to both national classification and IPC

**B. FIELDS SEARCHED**

Minimum documentation searched (classification system followed by classification symbols)
Int.Cl⁷  C12N9/16, C12N15/55, C07K16/40, A61K38/43

Documentation searched other than minimum documentation to the extent that such documents are included in the fields searched

Electronic data base consulted during the international search (name of data base and, where practicable, search terms used)
WPI, WPI/L, CAS ONLINE, BIOSIS PREVIEWS, DDBJ/EMBL/GenBank/Geneseq

**C. DOCUMENTS CONSIDERED TO BE RELEVANT**

| Category* | Citation of document, with indication, where appropriate, of the relevant passages | Relevant to claim No. |
|---|---|---|
| A | JP 10-201479 A (Toray Industries, Inc.), 04 August, 1998 (04.08.98)  (Family: none) | 1-17,19 |
| A | Henry Higgs et al., "Cloning of a Phosphatidic Acid-preferring Phospholopase A1 from Bovine Testis" J.Biol.Chem., Vol. 273 (1998) pp.5468-5477 | 1-17,19 |

☐ Further documents are listed in the continuation of Box C.　☐ See patent family annex.

| | |
|---|---|
| \* Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>26 September, 2000 (26.09.00) | Date of mailing of the international search report<br>24 October, 2000 (24.10.00) |
| Name and mailing address of the ISA/<br>Japanese Patent Office | Authorized officer |
| Facsimile No. | Telephone No. |

Form PCT/ISA/210 (second sheet) (July 1992)

**INTERNATIONAL SEARCH REPORT**

| International application No. |
| --- |
| PCT/JP00/04441 |

---

**Box I    Observations where certain claims were found unsearchable (Continuation of item 1 of first sheet)**

This international search report has not been established in respect of certain claims under Article 17(2)(a) for the following reasons:

1. ☒  Claims Nos.: 18
   because they relate to subject matter not required to be searched by this Authority, namely:

   Claim 18 involves methods for treatment of the human body.

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

**Box II    Observations where unity of invention is lacking (Continuation of item 2 of first sheet)**

This International Searching Authority found multiple inventions in this international application, as follows:

1. ☐  As all required additional search fees were timely paid by the applicant, this international search report covers all searchable claims.

2. ☐  As all searchable claims could be searched without effort justifying an additional fee, this Authority did not invite payment of any additional fee.

3. ☐  As only some of the required additional search fees were timely paid by the applicant, this international search report covers only those claims for which fees were paid, specifically claims Nos.:

4. ☐  No required additional search fees were timely paid by the applicant. Consequently, this international search report is restricted to the invention first mentioned in the claims; it is covered by claims Nos.:

**Remark on Protest**    ☐    The additional search fees were accompanied by the applicant's protest.
                         ☐    No protest accompanied the payment of additional search fees.

Form PCT/ISA/210 (continuation of first sheet (1)) (July 1992)